# EUROPEAN PATENT SPECIFICATION

(11) **EP 2 061 494 B1**
(45) Date of publication and mention of the grant of the patent: **01.02.2012**
(21) Application number: 06795477.6
(22) Date of filing: 08.09.2006
(51) Int. Cl.: A61K 38/17, A01K 67/027, C12N 15/86, G01N 33/50, C12N 5/10, A61P 35/00

(54) **REGULATION OF EXPRESSION OF P140CAP PROTEIN IN TUMORS**
REGULIERUNG DER EXPRESSION VON P140CAP-PROTEIN IN TUMOREN
RÉGULATION DE L'EXPRESSION DE LA PROTÉINE P140CAP DANS LES TUMEURS

(43) Date of publication of application: 27.05.2009
(73) Proprietor: Universita' Degli Studi di Torino, 10124 Torino (IT)
(72) Inventor: DEFILIPPI, Paola, I-10025 Pino Torinese (IT); DI STEFANO, Paola, I-10126 Torino (IT); SILENGO, Lorenzo, I-10134 Torino (IT)
(74) Representative: Freyria Fava, Cristina
(86) International application number: PCT/IB2006/002514
(87) International publication number: WO 2008/029189

(56) References cited:
- DI STEFANO PAOLA ET AL: "p130Cas-associated protein (p140Cap) as a new tyrosine-phosphorylated protein involved in cell spreading." MOLECULAR BIOLOGY OF THE CELL, vol. 15, no. 2, February 2004 (2004-02), pages 787-800, XP002432377 ISSN: 1059-1524 cited in the application
- DEFILIPPI ET AL: "p130Cas: a versatile scaffold in signaling networks" TRENDS IN CELL BIOLOGY, ELSEVIER SCIENCE LTD, XX, vol. 16, no. 5, May 2006 (2006-05), pages 257-263, XP005445130 ISSN: 0962-8924
- CHIN L S ET AL: "SNIP, a novel SNAP-25-interacting protein implicated in regulated exocytosis." THE JOURNAL OF BIOLOGICAL CHEMISTRY 14 JAN 2000, vol. 275, no. 2, 14 January 2000 (2000-01-14), pages 1191-1200, XP009083346 ISSN: 0021-9258

### SEQUENCE LISTING

<110> Università degli Studi di Torino
   <120> Regulation of expression of p140Cap protein in tumors
   <130> EWO8033-CF
   <160> 11
   <170> PatentIn version 3.3
   <210> 1
   <211> 1183
   <212> PRT
   <213> human
<400> 1
   Met Gly Asn Ala Pro Ser Gln Asp Pro Glu Arg Ser Ser Pro Pro Met
   1 5 10 15
   Leu Ser Ala Asp Asp Ala Glu Tyr Pro Arg Glu Tyr Arg Thr Leu Gly
   20 25 30
   Gly Gly Gly Gly Gly Gly Ser Gly Gly Arg Arg Phe Ser Asn Val Gly
   35 40 45
   Leu Val His Thr Ser Glu Arg Arg His Thr Val Ile Ala Ala Gln Ser
   50 55 60
   Leu Glu Ala Leu Ser Gly Leu Gln Lys Ala Asp Ala Asp Arg Lys Arg
   65 70 75 80
   Asp Ala Phe Met Asp His Leu Lys Ser Lys Tyr Pro Gln His Ala Leu
   85 90 95
   Ala Leu Arg Gly Gln Gln Asp Arg Met Arg Glu Gln Pro Asn Tyr Trp
   100 105 110
   Ser Phe Lys Thr Arg Ser Ser Arg His Thr Gln Gly Ala Gln Pro Gly
   115 120 125
   Leu Ala Asp Gln Ala Ala Lys Leu Ser Tyr Ala Ser Ala Glu Ser Leu
   130 135 140
   Glu Thr Met Ser Glu Ala Glu Leu Pro Leu Gly Phe Ser Arg Met Asn
   145 150 155 160
   Arg Phe Arg Gln Ser Leu Pro Leu Ser Arg Ser Ala Ser Gln Thr Lys
   165 170 175
   Pro Arg Ser Pro Gly Val Leu Phe Leu Gln Phe Gly Glu Glu Thr Arg
   180 185 190
   Arg Val His Ile Thr His Glu Val Ser Ser Leu Asp Thr Leu His Ala
   195 200 205
   Leu Ile Ala His Met Phe Pro Gln Lys Leu Thr Met Gly Met Leu Lys
   210 215 220
   Ser Pro Asn Thr Ala Ile Leu Ile Lys Asp Glu Ala Arg Asn Val Phe
   225 230 235 240
   Tyr Glu Leu Glu Asp Val Arg Asp Ile Gln Asp Arg Ser Ile Ile Lys
   245 250 255
   Ile Tyr Arg Lys Glu Pro Leu Tyr Ala Ala Phe Pro Gly Ser His Leu
   260 265 270
   Thr Asn Gly Asp Leu Arg Arg Glu Met Val Tyr Ala Ser Arg Glu Ser
   275 280 285
   Ser Pro Thr Arg Arg Leu Asn Asn Leu Ser Pro Ala Pro His Leu Ala
   290 295 300
   Ser Gly Ser Pro Pro Pro Gly Leu Pro Ser Gly Leu Pro Ser Gly Leu
   305 310 315 320
   Gln Ser Gly Ser Pro Ser Arg Ser Arg Leu Ser Tyr Ala Gly Gly Arg
   325 330 335
   Pro Pro Ser Tyr Ala Gly Ser Pro Val His His Ala Ala Glu Arg Leu
   340 345 350
   Gly Gly Ala Pro Ala Ala Gln Gly Val Ser Pro Ser Pro Ser Ala Ile
   355 360 365
   Leu Glu Arg Arg Asp Val Lys Pro Asp Glu Asp Leu Ala Ser Lys Ala
   370 375 380
   Gly Gly Met Val Leu Val Lys Gly Glu Gly Leu Tyr Ala Asp Pro Tyr
   385 390 395 400
   Gly Leu Leu His Glu Gly Arg Leu Ser Leu Ala Ala Ala Ala Gly Asp
   405 410 415
   Pro Phe Ala Tyr Pro Gly Ala Gly Gly Leu Tyr Lys Arg Gly Ser Val
   420 425 430
   Arg Ser Leu Ser Thr Tyr Ser Ala Ala Ala Leu Gln Ser Asp Leu Glu
   435 440 445
   Asp Ser Leu Tyr Lys Ala Ala Gly Gly Gly Gly Pro Leu Tyr Gly Asp
   450 455 460
   Gly Tyr Gly Phe Arg Leu Pro Pro Ser Ser Pro Gln Lys Leu Ala Asp
   465 470 475 480
   Val Ala Ala Pro Pro Gly Gly Pro Pro Pro Pro His Ser Pro Tyr Ser
   485 490 495
   Gly Pro Pro Ser Arg Gly Ser Pro Val Arg Gln Ser Phe Arg Lys Asp
   500 505 510
   Ser Gly Ser Ser Ser Val Phe Ala Glu Ser Pro Gly Gly Lys Thr Arg
   515 520 525
   Ser Ala Gly Ser Ala Ser Thr Ala Gly Ala Pro Pro Ser Glu Leu Phe
   530 535 540
   Pro Gly Pro Gly Glu Arg Ser Leu Val Gly Phe Gly Pro Pro Val Pro
   545 550 555 560
   Ala Lys Asp Thr Glu Thr Arg Glu Arg Met Glu Ala Met Glu Lys Gln
   565 570 575
   Ile Ala Ser Leu Thr Gly Leu Val Gln Ser Ala Leu Leu Arg Gly Ser
   580 585 590
   Glu Pro Glu Thr Pro Ser Glu Lys Ile Glu Gly Ser Asn Gly Ala Ala
   595 600 605
   Thr Pro Ser Ala Pro Cys Gly Ser Gly Gly Arg Ser Ser Gly Ala Thr
   610 615 620
   Pro Val Ser Gly Pro Pro Pro Pro Ser Ala Ser Ser Thr Pro Ala Gly
   625 630 635 640
   Gln Pro Thr Ala Val Ser Arg Leu Gln Met Gln Leu His Leu Arg Gly
   645 650 655
   Leu Gln Asn Ser Ala Ser Asp Leu Arg Gly Gln Leu Gln Gln Leu Arg
   660 665 670
   Lys Leu Gln Leu Gln Asn Gln Glu Ser Val Arg Ala Leu Leu Lys Arg
   675 680 685
   Thr Glu Ala Glu Leu Ser Met Arg Val Ser Glu Ala Ala Arg Arg Gln
   690 695 700
   Glu Asp Pro Leu Gln Arg Gln Arg Thr Leu Val Glu Glu Glu Arg Leu
   705 710 715 720
   Arg Tyr Leu Asn Asp Glu Glu Leu Ile Thr Gln Gln Leu Asn Asp Leu
   725 730 735
   Glu Lys Ser Val Glu Lys Ile Gln Arg Asp Val Ser His Asn His Arg
   740 745 750
   Leu Val Pro Gly Pro Glu Leu Glu Glu Lys Ala Leu Val Leu Lys Gln
   755 760 765
   Leu Gly Glu Thr Leu Thr Glu Leu Lys Ala His Phe Pro Gly Leu Gln
   770 775 780
   Ser Lys Met Arg Val Val Leu Arg Val Glu Val Glu Ala Val Lys Phe
   785 790 795 800
   Leu Lys Glu Glu Pro Gln Arg Leu Asp Gly Leu Leu Lys Arg Cys Arg
   805 810 815
   Gly Val Thr Asp Thr Leu Ala Gln Ile Arg Arg Gln Val Asp Glu Gly
   820 825 830
   Val Trp Pro Pro Pro Asn Asn Leu Leu Ser Gln Ser Pro Lys Lys Val
   835 840 845
   Thr Ala Glu Thr Asp Phe Asn Lys Ser Val Asp Phe Glu Met Pro Pro
   850 855 860
   Pro Ser Pro Pro Leu Asn Leu His Glu Leu Ser Gly Pro Ala Glu Gly
   865 870 875 880
   Ala Ser Leu Thr Pro Lys Gly Gly Asn Pro Thr Lys Gly Leu Asp Thr
   885 890 895
   Pro Gly Lys Arg Ser Val Asp Lys Ala Val Ser Val Glu Ala Ala Glu
   900 905 910
   Arg Asp Trp Glu Glu Lys Arg Ala Ala Leu Thr Gln Tyr Ser Ala Lys
   915 920 925
   Asp Ile Asn Arg Leu Leu Glu Glu Thr Gln Ala Glu Leu Leu Lys Ala
   930 935 940
   Ile Pro Asp Leu Asp Cys Ala Ser Lys Ala His Pro Gly Pro Ala Pro
   945 950 955 960
   Thr Pro Asp His Lys Pro Pro Lys Ala Pro His Gly Gln Lys Ala Ala
   965 970 975
   Pro Arg Thr Glu Pro Ser Gly Arg Arg Gly Ser Asp Glu Leu Thr Val
   980 985 990
   Pro Arg Tyr Arg Thr Glu Lys Pro Ser Lys Ser Pro Pro Pro Pro Pro
   995 1000 1005
   Pro Arg Arg Ser Phe Pro Ser Ser His Gly Leu Thr Thr Thr Arg
   1010 1015 1020
   Thr Gly Glu Val Val Val Thr Ser Lys Lys Asp Ser Ala Phe Ile
   1025 1030 1035
   Lys Lys Ala Glu Ser Glu Glu Leu Glu Val Gln Lys Pro Gln Val
   1040 1045 1050
   Lys Leu Arg Arg Ala Val Ser Glu Val Ala Arg Pro Ala Ser Thr
   1055 1060 1065
   Pro Pro Ile Met Ala Ser Ala Ile Lys Asp Glu Asp Asp Glu Asp
   1070 1075 1080
   Arg Ile Ile Ala Glu Leu Glu Ser Gly Gly Gly Ser Val Pro Pro
   1085 1090 1095
   Met Lys Val Val Thr Pro Gly Ala Ser Arg Leu Lys Ala Ala Gln
   1100 1105 1110
   Gly Gln Ala Gly Ser Pro Asp Lys Ser Lys His Gly Lys Gln Arg
   1115 1120 1125
   Ala Glu Tyr Met Arg Ile Gln Ala Gln Gln Gln Ala Thr Lys Pro
   1130 1135 1140
   Ser Lys Glu Met Ser Gly Ser Asn Glu Thr Ser Ser Pro Val Ser
   1145 1150 1155
   Glu Lys Pro Ser Ala Ser Arg Thr Ser Ile Pro Val Leu Thr Ser
   1160 1165 1170
   Phe Gly Ala Arg Asn Ser Ser Ile Ser Phe
   1175 1180
<210> 2
   <211> 1216
   <212> PRT
   <213> mouse
<400> 2
   Met Gly Asn Ala Pro Ser Gln Asp Pro Glu Arg Ser Ser Pro Pro Met
   1 5 10 15
   Leu Ser Ala Asp Asp Ala Glu Tyr Pro Arg Glu Tyr Arg Thr Leu Gly
   20 25 30
   Gly Gly Gly Gly Gly Gly Ser Gly Gly Arg Arg Phe Ser Asn Val Gly
   35 40 45
   Leu Val His Thr Ser Glu Arg Arg His Thr Val Ile Ala Ala Gln Ser
   50 55 60
   Leu Glu Ala Leu Ser Gly Leu Gln Lys Ala Asp Ala Asp Arg Lys Arg
   65 70 75 80
   Asp Ala Phe Met Asp His Leu Lys Ser Lys Tyr Pro Gln His Ala Leu
   85 90 95
   Ala Leu Arg Gly Gln Gln Asp Arg Met Arg Glu Gln Val Gly Gly Trp
   100 105 110
   Thr Val Asp Pro Val Cys Leu Leu Ser Ser Leu Cys Ser His Leu His
   115 120 125
   Gly Asp Ser Thr Pro Ser Gly Ala Gly Gln Pro Ala Gln Gln Pro Asn
   130 135 140
   Tyr Trp Ser Phe Lys Thr Arg Ser Ser Arg His Thr Gln Gly Ala Gln
   145 150 155 160
   Pro Gly Leu Ala Asp Gln Ala Ala Lys Leu Ser Tyr Ala Ser Ala Glu
   165 170 175
   Ser Leu Glu Thr Met Ser Glu Ala Glu Leu Pro Leu Gly Phe Ser Arg
   180 185 190
   Met Asn Arg Phe Arg Gln Ser Leu Pro Leu Ser Arg Ser Ala Ser Gln
   195 200 205
   Thr Lys Leu Arg Ser Pro Gly Val Leu Phe Leu Gln Phe Gly Glu Glu
   210 215 220
   Thr Arg Arg Val His Ile Thr His Glu Val Ser Ser Leu Asp Thr Leu
   225 230 235 240
   His Ala Leu Ile Ala His Met Phe Pro Gln Lys Leu Thr Met Gly Met
   245 250 255
   Leu Lys Ser Pro Asn Thr Ala Ile Leu Ile Lys Asp Glu Ala Arg Asn
   260 265 270
   Val Phe Tyr Glu Leu Glu Asp Val Arg Asp Ile Gln Asp Arg Ser Ile
   275 280 285
   Ile Lys Ile Tyr Arg Lys Glu Pro Leu Tyr Ala Ala Phe Pro Gly Ser
   290 295 300
   His Leu Thr Asn Gly Asp Leu Arg Arg Glu Met Val Tyr Ala Ser Arg
   305 310 315 320
   Glu Ser Ser Pro Thr Arg Arg Leu Asn Asn Leu Ser Pro Ala Ser His
   325 330 335
   Leu Ala Ser Ser Ser Pro Pro Pro Gly Leu Pro Ser Gly Leu Pro Ser
   340 345 350
   Gly Leu Pro Ser Gly Ser Pro Ser Arg Ser Arg Leu Ser Tyr Ala Gly
   355 360 365
   Gly Arg Pro Pro Ser Tyr Ala Gly Ser Pro Val His His Ala Ala Glu
   370 375 380
   Arg Leu Gly Gly Ala Pro Thr Gly Gln Gly Val Ser Pro Ser Pro Ser
   385 390 395 400
   Ala Ile Leu Glu Arg Arg Asp Val Lys Pro Asp Glu Asp Leu Ala Gly
   405 410 415
   Lys Ala Gly Gly Met Val Leu Val Lys Gly Glu Gly Leu Tyr Ala Asp
   420 425 430
   Pro Tyr Gly Leu Leu His Glu Gly Arg Leu Ser Leu Ala Ala Ala Ala
   435 440 445
   Glu Thr His Ser His Thr Arg Ala Arg Ala Ala Cys Thr Ser Gly Val
   450 455 460
   Pro Cys Ala Leu Ser Ala Pro Thr Pro Leu Pro Arg Cys Ser Pro Thr
   465                 470                 475                 480
   Trp Arg Thr Arg Cys Thr Arg Arg Ala Leu Ala Ala Leu Tyr Gly Asp
                   485                 490                 495
   Gly Tyr Gly Phe Arg Leu Pro Pro Ser Ser Pro Gln Lys Leu Ala Asp
               500                 505                 510
   Val Ser Ala Pro Ser Gly Gly Pro Pro Pro Pro His Ser Pro Tyr Ser
           515                 520                 525
   Gly Pro Pro Ser Arg Gly Ser Pro Val Arg Gln Ser Phe Arg Lys Asp
       530                 535                 540
   Ser Gly Ser Ser Ser Val Phe Ala Glu Ser Pro Gly Gly Lys Ala Arg
   545                 550                 555                 560
   Ser Thr Gly Ser Ala Ser Thr Ala Gly Ala Pro Pro Ser Glu Leu Phe
                   565                 570                 575
   Pro Gly Pro Gly Glu Arg Ser Leu Val Gly Phe Gly Pro Pro Val Pro
               580                 585                 590
   Ala Lys Asp Thr Glu Thr Arg Glu Arg Met Glu Ala Met Glu Lys Gln
           595                 600                 605
   Ile Ala Ser Leu Thr Gly Leu Val Gln Ser Ala Leu Leu Arg Gly Ser
       610                 615                 620
   Glu Pro Glu Thr Pro Ser Glu Lys Val Glu Gly Ser Asn Gly Ala Ala
   625                 630                 635                 640
   Thr Pro Ser Ala Pro Val Cys Gly Ser Gly Ser Lys Ser Ser Gly Ala
                   645                 650                 655
   Thr Pro Val Ser Gly Pro Pro Pro Pro Ser Ala Ser Ser Thr Pro Ala
               660                 665                 670
   Gly Gln Pro Thr Ala Val Ser Arg Leu Gln Met Gln Leu His Leu Arg
           675                 680                 685
   Gly Leu Gln Asn Ser Ala Ser Asp Leu Arg Gly Gln Leu Gln Gln Leu
       690                 695                 700
   Arg Asn Val Gln Leu Gln Asn Gln Glu Ser Val Arg Ala Leu Leu Lys
   705                 710                 715                 720
   Pro Thr Glu Ala Asp Val Ser Met Arg Val Ser Glu Ala Ala Arg Arg
                   725                 730                 735
   Gln Glu Asp Pro Leu Gln Arg Gln Arg Thr Leu Val Glu Glu Glu Arg
               740                 745                 750
   Leu Arg Tyr Leu Asn Asp Glu Glu Leu Ile Thr Gln Gln Leu Asn Asp
           755                 760                 765
   Leu Glu Lys Ser Val Glu Lys Ile Gln Arg Asp Val Ala His Asn His
   770 775 780
   Arg Leu Met Pro Gly Pro Glu Leu Glu Glu Lys Ala Leu Val Leu Lys
   785 790 795 800
   Gln Leu Gly Glu Thr Leu Thr Glu Leu Lys Ala His Phe Pro Gly Leu
   805 810 815
   Gln Ser Lys Met Arg Val Val Leu Arg Val Glu Val Glu Ala Val Lys
   820 825 830
   Phe Leu Lys Glu Glu Pro Gln Arg Leu Asp Gly Leu Leu Lys Arg Cys
   835 840 845
   Arg Gly Val Thr Asp Thr Leu Ala Gln Ile Arg Arg Gln Val Asp Glu
   850 855 860
   Gly Met Trp Pro Pro Pro Asn Asn Leu Leu Asn Gln Ser Pro Lys Lys
   865 870 875 880
   Val Ala Ala Glu Thr Asp Phe Ser Lys Gly Leu Asp Phe Glu Ile Pro
   885 890 895
   Pro Pro Ser Pro Pro Leu Asn Leu His Glu Leu Ser Gly Pro Ala Glu
   900 905 910
   Gly Thr Pro Leu Thr Pro Lys Ser Thr Asn Pro Thr Lys Cys Leu Asp
   915 920 925
   Ala Ser Ser Lys Arg Asn Thr Asp Lys Ala Val Ser Val Glu Ala Ala
   930 935 940
   Glu Arg Asp Trp Glu Glu Lys Arg Ala Ala Leu Thr Gln Tyr Ser Ala
   945 950 955 960
   Lys Asp Ile Asn Arg Leu Leu Glu Glu Thr Gln Ala Glu Leu Leu Lys
   965 970 975
   Ala Ile Pro Asp Leu Asp Cys Ala Ser Lys Thr His Pro Gly Pro Ala
   980 985 990
   Pro Thr Pro Asp His Lys Pro Pro Lys Ala Pro His Gly Gln Lys Ala
   995 1000 1005
   Ala Pro Arg Thr Glu Pro Ser Gly Arg Arg Gly Ser Asp Glu Leu
   1010 1015 1020
   Thr Val Pro Arg Tyr Arg Thr Glu Lys Pro Ser Lys Ser Pro Pro
   1025 1030 1035
   Pro Pro Pro Pro Arg Arg Ser Phe Pro Ser Ser His Gly Leu Thr
   1040 1045 1050
   Thr Thr Arg Thr Gly Glu Val Val Val Thr Ser Lys Lys Asp Ser
   1055 1060 1065
   Val Phe Ile Lys Lys Ala Glu Ser Glu Glu Leu Glu Val Gln Lys
   1070 1075 1080
   Pro Gln Val Lys Leu Arg Arg Ala Val Ser Glu Val Val Arg Pro
   1085 1090 1095
   Ala Ser Thr Pro Pro Ile Met Ala Ser Ala Ile Lys Asp Glu Asp
   1100 1105 1110
   Asp Glu Glu Arg Ile Ile Ala Glu Leu Glu Ser Gly Gly Ser Ser
   1115 1120 1125
   Val Pro Pro Met Lys Val Val Thr Pro Gly Ala Ser Arg Leu Lys
   1130 1135 1140
   Ala Ala Gln Gly Pro Ala Gly Ser Pro Asp Lys Gly Lys His Gly
   1145 1150 1155
   Lys Gln Arg Thr Glu Tyr Met Arg Ile Gln Ala Gln Gln Gln Ala
   1160 1165 1170
   Thr Lys Pro Ser Lys Glu Val Ser Gly Pro Asn Glu Thr Ser Ser
   1175 1180 1185
   Pro Gly Ser Glu Lys Pro Ser Gly Ser Arg Thr Ser Ile Pro Val
   1190 1195 1200
   Leu Thr Ser Phe Gly Ala Arg Asn Ser Ser Ile Ser Phe
   1205 1210 1215
<210> 3
   <211> 47
   <212> DNA
   <213> mouse
<400> 3
   cggcagagac tgacttcaat ctcttgaatt gaagtcagtc tctgccg 47
<210> 4
   <211> 770
   <212> PRT
   <213> mouse
<400> 4
   Met Gly Asn Ala Pro Ser Gln Asp Pro Glu Arg Ser Ser Pro Pro Met
   1 5 10 15
   Leu Ser Ala Asp Asp Ala Glu Tyr Pro Arg Glu Tyr Arg Thr Leu Gly
   20 25 30
   Gly Gly Gly Gly Gly Gly Ser Gly Gly Arg Arg Phe Ser Asn Val Gly
   35 40 45
   Leu Val His Thr Ser Glu Arg Arg His Thr Val Ile Ala Ala Gln Ser
   50 55 60
   Leu Glu Ala Leu Ser Gly Leu Gln Lys Ala Asp Ala Asp Arg Lys Arg
   65 70 75 80
   Asp Ala Phe Met Asp His Leu Lys Ser Lys Tyr Pro Gln His Ala Leu
   85 90 95
   Ala Leu Arg Gly Gln Gln Asp Arg Met Arg Glu Gln Val Gly Gly Trp
   100 105 110
   Thr Val Asp Pro Val Cys Leu Leu Ser Ser Leu Cys Ser His Leu His
   115 120 125
   Gly Asp Ser Thr Pro Ser Gly Ala Gly Gln Pro Ala Gln Gln Pro Asn
   130 135 140
   Tyr Trp Ser Phe Lys Thr Arg Ser Ser Arg His Thr Gln Gly Ala Gln
   145 150 155 160
   Pro Gly Leu Ala Asp Gln Ala Ala Lys Leu Ser Tyr Ala Ser Ala Glu
   165 170 175
   Ser Leu Glu Thr Met Ser Glu Ala Glu Leu Pro Leu Gly Phe Ser Arg
   180 185 190
   Met Asn Arg Phe Arg Gln Ser Leu Pro Leu Ser Arg Ser Ala Ser Gln
   195 200 205
   Thr Lys Leu Arg Ser Pro Gly Val Leu Phe Leu Gln Phe Gly Glu Glu
   210 215 220
   Thr Arg Arg Val His Ile Thr His Glu Val Ser Ser Leu Asp Thr Leu
   225 230 235 240
   His Ala Leu Ile Ala His Met Phe Pro Gln Lys Leu Thr Met Gly Met
   245 250 255
   Leu Lys Ser Pro Asn Thr Ala Ile Leu Ile Lys Asp Glu Ala Arg Asn
   260 265 270
   Val Phe Tyr Glu Leu Glu Asp Val Arg Asp Ile Gln Asp Arg Ser Ile
   275 280 285
   Ile Lys Ile Tyr Arg Lys Glu Pro Leu Tyr Ala Ala Phe Pro Gly Ser
   290 295 300
   His Leu Thr Asn Gly Asp Leu Arg Arg Glu Met Val Tyr Ala Ser Arg
   305 310 315 320
   Glu Ser Ser Pro Thr Arg Arg Leu Asn Asn Leu Ser Pro Ala Ser His
   325 330 335
   Leu Ala Ser Ser Ser Pro Pro Pro Gly Leu Pro Ser Gly Leu Pro Ser
               340                 345                 350
   Gly Leu Pro Ser Gly Ser Pro Ser Arg Ser Arg Leu Ser Tyr Ala Gly
           355                 360                 365
   Gly Arg Pro Pro Ser Tyr Ala Gly Ser Pro Val His His Ala Ala Glu
       370                 375                 380
   Arg Leu Gly Gly Ala Pro Thr Gly Gln Gly Val Ser Pro Ser Pro Ser
   385                 390                 395                 400
   Ala Ile Leu Glu Arg Arg Asp Val Lys Pro Asp Glu Asp Leu Ala Gly
                   405                 410                 415
   Lys Ala Gly Gly Met Val Leu Val Lys Gly Glu Gly Leu Tyr Ala Asp
               420                 425                 430
   Pro Tyr Gly Leu Leu His Glu Gly Arg Leu Ser Leu Ala Ala Ala Ala
           435                 440                 445
   Glu Thr His Ser His Thr Arg Ala Arg Ala Ala Cys Thr Ser Gly Val
       450                 455                 460
   Pro Cys Ala Leu Ser Ala Pro Thr Pro Leu Pro Arg Cys Ser Pro Thr
   465                 470                 475                 480
   Trp Arg Thr Arg Cys Thr Arg Arg Ala Leu Ala Ala Leu Tyr Gly Asp
                   485                 490                 495
   Gly Tyr Gly Phe Arg Leu Pro Pro Ser Ser Pro Gln Lys Leu Ala Asp
               500                 505                 510
   Val Ser Ala Pro Ser Gly Gly Pro Pro Pro Pro His Ser Pro Tyr Ser
           515                 520                 525
   Gly Pro Pro Ser Arg Gly Ser Pro Val Arg Gln Ser Phe Arg Lys Asp
       530                 535                 540
   Ser Gly Ser Ser Ser Val Phe Ala Glu Ser Pro Gly Gly Lys Ala Arg
   545                 550                 555                 560
   Ser Thr Gly Ser Ala Ser Thr Ala Gly Ala Pro Pro Ser Glu Leu Phe
                   565                 570                 575
   Pro Gly Pro Gly Glu Arg Ser Leu Val Gly Phe Gly Pro Pro Val Pro
               580                 585                 590
   Ala Lys Asp Thr Glu Thr Arg Glu Arg Met Glu Ala Met Glu Lys Gln
           595                 600                 605
   Ile Ala Ser Leu Thr Gly Leu Val Gln Ser Ala Leu Leu Arg Gly Ser
       610                 615                 620
   Glu Pro Glu Thr Pro Ser Glu Lys Val Glu Gly Ser Asn Gly Ala Ala
   625                 630                 635                 640
   Thr Pro Ser Ala Pro Val Cys Gly Ser Gly Ser Lys Ser Ser Gly Ala
                   645                 650                 655
   Thr Pro Val Ser Gly Pro Pro Pro Pro Ser Ala Ser Ser Thr Pro Ala
               660                 665                 670
   Gly Gln Pro Thr Ala Val Ser Arg Leu Gln Met Gln Leu His Leu Arg
           675                 680                 685
   Gly Leu Gln Asn Ser Ala Ser Asp Leu Arg Gly Gln Leu Gln Gln Leu
       690                 695                 700
   Arg Asn Val Gln Leu Gln Asn Gln Glu Ser Val Arg Ala Leu Leu Lys
   705                 710                 715                 720
   Pro Thr Glu Ala Asp Val Ser Met Arg Val Ser Glu Ala Ala Arg Arg
                   725                 730                 735
   Gln Glu Asp Pro Leu Gln Arg Gln Arg Thr Leu Val Glu Glu Glu Arg
               740                 745                 750
   Leu Arg Tyr Leu Asn Asp Glu Glu Leu Ile Thr Gln Gln Leu Asn Asp
           755                 760                 765
   Leu Glu
       770
<210> 5
   <211> 105
   <212> PRT
   <213> mouse
<400> 5
   Gln Ala Glu Glu Trp Tyr Phe Gly Lys Ile Thr Arg Arg Glu Ser Glu
   1               5                   10                  15
   Arg Leu Leu Leu Asn Ala Glu Asn Pro Arg Gly Thr Phe Leu Val Arg
               20                  25                  30
   Glu Ser Glu Thr Thr Lys Gly Ala Tyr Cys Leu Ser Val Ser Asp Phe
           35                  40                  45
   Asp Asn Ala Lys Gly Leu Asn Val Lys His Tyr Lys Ile Arg Lys Leu
       50                  55                  60
   Asp Ser Gly Gly Phe Tyr Ile Thr Ser Arg Thr Gln Phe Asn Ser Leu
   65                  70                  75                  80
   Gln Gln Leu Val Ala Tyr Tyr Ser Lys His Ala Asp Gly Leu Cys His
                   85                  90                  95
   Arg Leu Thr Thr Val Cys Pro Thr Ser
   100 105
<210> 6
   <211> 60
   <212> PRT
   <213> mouse
<400> 6
   Thr Thr Phe Val Ala Leu Tyr Asp Tyr Glu Ser Arg Thr Glu Thr Asp
   1 5 10 15
   Leu Ser Phe Lys Lys Gly Glu Arg Leu Gln Ile Val Asn Asn Thr Glu
   20 25 30
   Gly Asp Trp Trp Leu Ala His Ser Leu Ser Thr Gly Gln Thr Gly Tyr
   35 40 45
   Ile Pro Ser Asn Tyr Val Ala Pro Ser Asp Ser Ile
   50 55 60
<210> 7
   <211> 305
   <212> PRT
   <213> mouse
<400> 7
   Met Gly Asn Ala Pro Ser Gln Asp Pro Glu Arg Ser Ser Pro Pro Met
   1 5 10 15
   Leu Ser Ala Asp Asp Ala Glu Tyr Pro Arg Glu Tyr Arg Thr Leu Gly
   20 25 30
   Gly Gly Gly Gly Gly Gly Ser Gly Gly Arg Arg Phe Ser Asn Val Gly
   35 40 45
   Leu Val His Thr Ser Glu Arg Arg His Thr Val Ile Ala Ala Gln Ser
   50 55 60
   Leu Glu Ala Leu Ser Gly Leu Gln Lys Ala Asp Ala Asp Arg Lys Arg
   65 70 75 80
   Asp Ala Phe Met Asp His Leu Lys Ser Lys Tyr Pro Gln His Ala Leu
   85 90 95
   Ala Leu Arg Gly Gln Gln Asp Arg Met Arg Glu Gln Val Gly Gly Trp
   100 105 110
   Thr Val Asp Pro Val Cys Leu Leu Ser Ser Leu Cys Ser His Leu His
   115 120 125
   Gly Asp Ser Thr Pro Ser Gly Ala Gly Gln Pro Ala Gln Gln Pro Asn
   130 135 140
   Tyr Trp Ser Phe Lys Thr Arg Ser Ser Arg His Thr Gln Gly Ala Gln
   145 150 155 160
   Pro Gly Leu Ala Asp Gln Ala Ala Lys Leu Ser Tyr Ala Ser Ala Glu
   165 170 175
   Ser Leu Glu Thr Met Ser Glu Ala Glu Leu Pro Leu Gly Phe Ser Arg
   180 185 190
   Met Asn Arg Phe Arg Gln Ser Leu Pro Leu Ser Arg Ser Ala Ser Gln
   195 200 205
   Thr Lys Leu Arg Ser Pro Gly Val Leu Phe Leu Gln Phe Gly Glu Glu
   210 215 220
   Thr Arg Arg Val His Ile Thr His Glu Val Ser Ser Leu Asp Thr Leu
   225 230 235 240
   His Ala Leu Ile Ala His Met Phe Pro Gln Lys Leu Thr Met Gly Met
   245 250 255
   Leu Lys Ser Pro Asn Thr Ala Ile Leu Ile Lys Asp Glu Ala Arg Asn
   260 265 270
   Val Phe Tyr Glu Leu Glu Asp Val Arg Asp Ile Gln Asp Arg Ser Ile
   275 280 285
   Ile Lys Ile Tyr Arg Lys Glu Pro Leu Tyr Ala Ala Phe Pro Gly Ser
   290 295 300
   His
   305
<210> 8
   <211> 263
   <212> PRT
   <213> mouse
<400> 8
   His Leu Thr Asn Gly Asp Leu Arg Arg Glu Met Val Tyr Ala Ser Arg
   1 5 10 15
   Glu Ser Ser Pro Thr Arg Arg Leu Asn Asn Leu Ser Pro Ala Ser His
   20 25 30
   Leu Ala Ser Ser Ser Pro Pro Pro Gly Leu Pro Ser Gly Leu Pro Ser
   35 40 45
   Gly Leu Pro Ser Gly Ser Pro Ser Arg Ser Arg Leu Ser Tyr Ala Gly
   50 55 60
   Gly Arg Pro Pro Ser Tyr Ala Gly Ser Pro Val His His Ala Ala Glu
   65 70 75 80
   Arg Leu Gly Gly Ala Pro Thr Gly Gln Gly Val Ser Pro Ser Pro Ser
   85 90 95
   Ala Ile Leu Glu Arg Arg Asp Val Lys Pro Asp Glu Asp Leu Ala Gly
   100 105 110
   Lys Ala Gly Gly Met Val Leu Val Lys Gly Glu Gly Leu Tyr Ala Asp
   115 120 125
   Pro Tyr Gly Leu Leu His Glu Gly Arg Leu Ser Leu Ala Ala Ala Ala
   130 135 140
   Glu Thr His Ser His Thr Arg Ala Arg Ala Ala Cys Thr Ser Gly Val
   145 150 155 160
   Pro Cys Ala Leu Ser Ala Pro Thr Pro Leu Pro Arg Cys Ser Pro Thr
   165 170 175
   Trp Arg Thr Arg Cys Thr Arg Arg Ala Leu Ala Ala Leu Tyr Gly Asp
   180 185 190
   Gly Tyr Gly Phe Arg Leu Pro Pro Ser Ser Pro Gln Lys Leu Ala Asp
   195 200 205
   Val Ser Ala Pro Ser Gly Gly Pro Pro Pro Pro His Ser Pro Tyr Ser
   210 215 220
   Gly Pro Pro Ser Arg Gly Ser Pro Val Arg Gln Ser Phe Arg Lys Asp
   225 230 235 240
   Ser Gly Ser Ser Ser Val Phe Ala Glu Ser Pro Gly Gly Lys Ala Arg
   245 250 255
   Ser Thr Gly Ser Ala Ser Thr
   260
<210> 9
   <211> 220
   <212> PRT
   <213> mouse
<400> 9
   Thr Ala Gly Ala Pro Pro Ser Glu Leu Phe Pro Gly Pro Gly Glu Arg
   1 5 10 15
   Ser Leu Val Gly Phe Gly Pro Pro Val Pro Ala Lys Asp Thr Glu Thr
   20 25 30
   Arg Glu Arg Met Glu Ala Met Glu Lys Gln Ile Ala Ser Leu Thr Gly
   35 40 45
   Leu Val Gln Ser Ala Leu Leu Arg Gly Ser Glu Pro Glu Thr Pro Ser
       50                  55                  60
   Glu Lys Val Glu Gly Ser Asn Gly Ala Ala Thr Pro Ser Ala Pro Val
   65                  70                  75                  80
   Cys Gly Ser Gly Ser Lys Ser Ser Gly Ala Thr Pro Val Ser Gly Pro
                   85                  90                  95
   Pro Pro Pro Ser Ala Ser Ser Thr Pro Ala Gly Gln Pro Thr Ala Val
               100                 105                 110
   Ser Arg Leu Gln Met Gln Leu His Leu Arg Gly Leu Gln Asn Ser Ala
           115                 120                 125
   Ser Asp Leu Arg Gly Gln Leu Gln Gln Leu Arg Asn Val Gln Leu Gln
       130                 135                 140
   Asn Gln Glu Ser Val Arg Ala Leu Leu Lys Pro Thr Glu Ala Asp Val
   145                 150                 155                 160
   Ser Met Arg Val Ser Glu Ala Ala Arg Arg Gln Glu Asp Pro Leu Gln
                   165                 170                 175
   Arg Gln Arg Thr Leu Val Glu Glu Glu Arg Leu Arg Tyr Leu Asn Asp
               180                 185                 190
   Glu Glu Leu Ile Thr Gln Gln Leu Asn Asp Leu Glu Lys Ser Val Glu
           195                 200                 205
   Lys Ile Gln Arg Asp Val Ala His Asn His Arg Leu
       210                 215                 220
<210> 10
   <211> 212
   <212> PRT
   <213> mouse
<400> 10
   Leu Met Pro Gly Pro Glu Leu Glu Glu Lys Ala Leu Val Leu Lys Gln
   1               5                   10                  15
   Leu Gly Glu Thr Leu Thr Glu Leu Lys Ala His Phe Pro Gly Leu Gln
               20                  25                  30
   Ser Lys Met Arg Val Val Leu Arg Val Glu Val Glu Ala Val Lys Phe
           35                  40                  45
   Leu Lys Glu Glu Pro Gln Arg Leu Asp Gly Leu Leu Lys Arg Cys Arg
       50                  55                  60
   Gly Val Thr Asp Thr Leu Ala Gln Ile Arg Arg Gln Val Asp Glu Gly
   65                  70                  75                  80
   Met Trp Pro Pro Pro Asn Asn Leu Leu Asn Gln Ser Pro Lys Lys Val
                   85                  90                  95
   Ala Ala Glu Thr Asp Phe Ser Lys Gly Leu Asp Phe Glu Ile Pro Pro
               100                 105                 110
   Pro Ser Pro Pro Leu Asn Leu His Glu Leu Ser Gly Pro Ala Glu Gly
           115                 120                 125
   Thr Pro Leu Thr Pro Lys Ser Thr Asn Pro Thr Lys Cys Leu Asp Ala
       130                 135                 140
   Ser Ser Lys Arg Asn Thr Asp Lys Ala Val Ser Val Glu Ala Ala Glu
   145                 150                 155                 160
   Arg Asp Trp Glu Glu Lys Arg Ala Ala Leu Thr Gln Tyr Ser Ala Lys
                   165                 170                 175
   Asp Ile Asn Arg Leu Leu Glu Glu Thr Gln Ala Glu Leu Leu Lys Ala
               180                 185                 190
   Ile Pro Asp Leu Asp Cys Ala Ser Lys Thr His Pro Gly Pro Ala Pro
           195                 200                 205
   Thr Pro Asp His
       210
<210> 11
   <211> 220
   <212> PRT
   <213> mouse
<400> 11
   His Lys Pro Pro Lys Ala Pro His Gly Gln Lys Ala Ala Pro Arg Thr
   1               5                   10                  15
   Glu Pro Ser Gly Arg Arg Gly Ser Asp Glu Leu Thr Val Pro Arg Tyr
               20                  25                  30
   Arg Thr Glu Lys Pro Ser Lys Ser Pro Pro Pro Pro Pro Pro Arg Arg
           35                  40                  45
   Ser Phe Pro Ser Ser His Gly Leu Thr Thr Thr Arg Thr Gly Glu Val
       50                  55                  60
   Val Val Thr Ser Lys Lys Asp Ser Val Phe Ile Lys Lys Ala Glu Ser
   65                  70                  75                  80
   Glu Glu Leu Glu Val Gln Lys Pro Gln Val Lys Leu Arg Arg Ala Val
                   85                  90                  95
   Ser Glu Val Val Arg Pro Ala Ser Thr Pro Pro Ile Met Ala Ser Ala
               100                 105                 110
   Ile Lys Asp Glu Asp Asp Glu Glu Arg Ile Ile Ala Glu Leu Glu Ser
   115 120 125
   Gly Gly Ser Ser Val Pro Pro Met Lys Val Val Thr Pro Gly Ala Ser
   130 135 140
   Arg Leu Lys Ala Ala Gln Gly Pro Ala Gly Ser Pro Asp Lys Gly Lys
   145 150 155 160
   His Gly Lys Gln Arg Thr Glu Tyr Met Arg Ile Gln Ala Gln Gln Gln
   165 170 175
   Ala Thr Lys Pro Ser Lys Glu Val Ser Gly Pro Asn Glu Thr Ser Ser
   180 185 190
   Pro Gly Ser Glu Lys Pro Ser Gly Ser Arg Thr Ser Ile Pro Val Leu
   195 200 205
   Thr Ser Phe Gly Ala Arg Asn Ser Ser Ile Ser Phe
   210 215 220
<210> 12
   <211> 19
   <212> DNA
   <213> mouse
   <220>
   <223> RNA interference down-regulating p140Cap expression
<400> 12
   cggcagagac tgacttcaa 19
<210> 13
   <211> 450
   <212> PRT
   <213> mouse
<400> 13
   Met Ser Ala Ile Gln Ala Ala Trp Pro Ser Gly Thr Glu Cys Ile Ala
   1 5 10 15
   Lys Tyr Asn Phe His Gly Thr Ala Glu Gln Asp Leu Pro Phe Cys Lys
   20 25 30
   Gly Asp Val Leu Thr Ile Val Ala Val Thr Lys Asp Pro Asn Trp Tyr
   35 40 45
   Lys Ala Lys Asn Lys Val Gly Arg Glu Gly Ile Ile Pro Ala Asn Tyr
   50 55 60
   Val Gln Lys Arg Glu Gly Val Lys Ala Gly Thr Lys Leu Ser Leu Met
   65 70 75 80
   Pro Trp Phe His Gly Lys Ile Thr Arg Glu Gln Ala Glu Arg Leu Leu
                       85                  90                  95
   Tyr Pro Pro Glu Thr Gly Leu Phe Leu Val Arg Glu Ser Thr Asn Tyr
               100                 105                 110
   Pro Gly Asp Tyr Thr Leu Cys Val Ser Cys Asp Gly Lys Val Glu His
           115                 120                 125
   Tyr Arg Ile Met Tyr His Ala Ser Lys Leu Ser Ile Asp Glu Glu Val
       130                 135                 140
   Tyr Phe Glu Asn Leu Met Gln Leu Val Glu His Tyr Thr Ser Asp Ala
   145                 150                 155                 160
   Asp Gly Leu Cys Thr Arg Leu Ile Lys Pro Lys Val Met Glu Gly Thr
                   165                 170                 175
   Val Ala Ala Gln Asp Glu Phe Tyr Arg Ser Gly Trp Ala Leu Asn Met
               180                 185                 190
   Lys Glu Leu Lys Leu Leu Gln Thr Ile Gly Lys Gly Glu Phe Gly Asp
           195                 200                 205
   Val Met Leu Gly Asp Tyr Arg Gly Asn Arg Val Ala Val Lys Cys Ile
       210                 215                 220
   Lys Asn Asp Ala Thr Ala Gln Ala Phe Leu Ala Glu Ala Ser Val Met
   225                 230                 235                 240
   Thr Gln Leu Arg His Ser Asn Leu Val Gln Leu Leu Gly Val Ile Val
                   245                 250                 255
   Glu Glu Lys Gly Gly Leu Tyr Ile Val Thr Glu Tyr Met Ala Lys Gly
               260                 265                 270
   Ser Leu Val Asp Tyr Leu Arg Ser Arg Gly Arg Ser Val Leu Gly Gly
           275                 280                 285
   Asp Cys Leu Leu Lys Phe Ser Leu Asp Val Cys Glu Ala Met Glu Tyr
       290                 295                 300
   Leu Glu Gly Asn Asn Phe Val His Arg Asp Leu Ala Ala Arg Asn Val
   305                 310                 315                 320
   Leu Val Ser Glu Asp Asn Val Ala Lys Val Ser Asp Phe Gly Leu Thr
                   325                 330                 335
   Lys Glu Ala Ser Ser Thr Gln Asp Thr Gly Lys Leu Pro Val Lys Trp
               340                 345                 350
   Thr Ala Pro Glu Ala Leu Arg Glu Lys Lys Phe Ser Thr Lys Ser Asp
           355                 360                 365
   Val Trp Ser Phe Gly Ile Leu Leu Trp Glu Ile Tyr Ser Phe Gly Arg
       370                 375                 380
   Val Pro Tyr Pro Arg Ile Pro Leu Lys Asp Val Val Pro Arg Val Glu
   385 390 395 400
   Lys Gly Tyr Lys Met Asp Ala Pro Asp Gly Cys Pro Pro Ala Val Tyr
   405 410 415
   Glu Val Met Lys Asn Cys Trp His Leu Asp Ala Ala Met Arg Pro Ser
   420 425 430
   Phe Leu Gln Leu Arg Glu Gln Leu Glu His Ile Lys Thr His Glu Leu
   435 440 445
   His Leu
   450
<210> 14
   <211> 18
   <212> DNA
   <213> artificial
   <220>
   <223> amplification primer
<400> 14
   cgcagcaccg ggagtgcc 18
<210> 15
   <211> 22
   <212> DNA
   <213> artificial
   <220>
   <223> amplification primer
<400> 15
   caactcagga cccggcatta gc 22
<210> 16
   <211> 7
   <212> PRT
   <213> mouse
<400> 16
   Pro Pro Pro Pro Pro Arg Arg
   1 5

## Description

### Technical field of the invention

The present invention relates to the area of p140Cap protein. More particularly, the present invention relates to the expression of human p140Cap in tumors and its regulation.

### Background of the invention

Signalling originated from the integrin family of cell-matrix receptors is central to many physiological and pathological processes such as embryogenesis, inflammatory response, tissue repair and cancer cell progression. Early integrin signalling induces activation of Focal adhesion kinase (FAK), Src family kinases and Rho family GTPases (Burridge and Wennerberg, 2004; Cabodi, 2005; Damsky and Ilic, 2002; Frame et al., 2002; Giancotti, 2003; Miranti and Brugge, 2002; Schwartz and Ginsberg, 2002). Upon integrin-mediated adhesion, Src kinase regulates cell migration through increased phosphorylation of Focal Adhesion Kinase (FAK) as well as other key adaptor molecules, like p130Cas (Defilippi, 2006; Mitra et al., 2005). Upon tyrosine phosphorylation (Goldberg et al., 2003; Shin et al., 2004), p130Cas recruits other proteins such as Crk and DOCK180 that regulate Rac activation, which is crucial for actin cytoskeleton organization and cell motility (Chodniewicz and Klemke, 2004). Cells derived from mice deficient in the three members of the Src family (Src/Yes/Fyn), FAK or p130Cas consistently exhibit impaired cell spreading and migration (Ilic et al., 1998; Ilic et al., 1995; Klinghoffer et al., 1999). On the other hand, in transformed cells, increased Src activity induces reorganization of epithelial adhesion systems and the actin cytoskeleton, leading to cell scattering and epithelial-mesenchymal transition (EMT) (Avizienyte et al., 2002; Boyer et al., 1997).
Aberrant activation of Src dramatically increases in vitro cell proliferation and in vivo tumour cell growth and invasion (Ishizawar and Parsons, 2004; Yeatman, 2004). Indeed, in transgenic mice over-expressing Src in the skin, squamous cell carcinomas develop more rapidly and with greater metastatic potential upon chemical carcinogenesis treatment (Matsumoto et al., 2003). Moreover Src activity has been found elevated during human epithelial cancer progression (Irby and Yeatman, 2000). For instance, Src tyrosine kinase activity is up-regulated in 70% of human breast tumor specimens, compared to normal breast epithelium (reviewed in (Biscardi et al., 2000)).

Increased Src activity induces reorganization of epithelial adhesion systems and actin cytoskeleton leading to "scattering" and a migratory phenotype of both normal and tumour-derived epithelial cells (Boyer et al., 1997). Accordingly, expression in rat carcinoma cells of the Src natural inhibitor Csk or of a dominant-negative mutant of Src dramatically reduces the number of in vivo metastases, confirming that Src confers a selective advantage during the metastatic process (Boyer et al., 1997). It is therefore important to understand the mechanisms by which Src induces migration and invasion in order to develop inhibitory strategies that might suppress tumorigenic properties.
We recently identified p140Cap as a novel adaptor protein indirectly associated to p130Cas (Di Stefano et al., 2004). p140Cap codistributes with cortical actin and actin stress fibers but not with focal adhesions. Interestingly expression of p140Cap in NIH3T3 and in ECV304 cells delays the onset of cell spreading in the early phases of cell adhesion to fibronectin. Moreover it is tyrosine phosphorylated upon integrin-dependent adhesion or EGF treatment, indicating a potential role as a downstream effector of cell-matrix and growth factor signalling (Di Stefano et al., 2004). p140Cap is expressed in the mammary gland and in breast cancer cells such as MCF7, T47D, MDA-MB-231 and MDA-MB-435. By loss and gain of function approaches, we show that p140Cap inhibits breast cancer cell motility, invasion and "in vivo" tumor growth and is a novel Src interacting protein that acts as a negative regulator of Src via activation of the Csk kinase.

### Summary of the invention

The present invention is defined in the claims and relates to the use of p140Cap protein for the manufacture of a medicament for treatment and prevention of human cancer pathologies; in particular. Herein described is also the use of i) p140Cap protein, peptides, fragments and/or derivatives thereof, and/or ii) nucleic acids encoding for p140Cap protein, peptides, fragments and/or derivatives thereof for the preparation of pharmaceutical compositions for the treatment of tumors and/or metastases in a subject suffering from a tumor, preferably a solid tumor.

Also described is a vector comprising at least a portion of the nucleotide sequence encoding p140Cap protein, wherein the vector is able to express p140Cap protein or fragments thereof. Still object of the present invention is the use of a vector comprising at least a portion of the nucleotide sequence encoding p140Cap protein for the preparation of a medicament for the treatment of tumors and metastases in a subject affected by a tumor.

Also described are reagents and methods for regulating expression, function and/or activity of human p140Cap protein. The use of p140Cap protein, and fragments thereof and/or the polynucleotide encoding for p140Cap protein and fragments thereof for the development of therapeutical approaches for treatment of cancer and, in particular, for screening for substances the treatment of patients suffering from a tumor is also described. In a preferred embodiment of the invention the tumor is a solid tumor.

Also described herein is a diagnostic method for determining the expression of p140Cap protein in cancer cells, and correlating such an expression with the prognosis of the disease.

The invention concerns also non-human transgenic animals as defined in the claims as model study for human pathologies, being transgenic for having altered p140Cap and Neu-T expression. Preferably the human pathology is cancer.

It is a further object of the invention cells derivable from the non-human transgenic animal of the invention. Different uses of the cells for the selection of molecules pharmacologically effective in stimulating over-expression of p140Cap gene are described.

According to the present invention, said objects are achieved thanks to the solution having the characteristics referred to specifically in the ensuing claims. The claims form integral part of the technical teaching herein provided in relation to the present invention.

### Description of the drawings

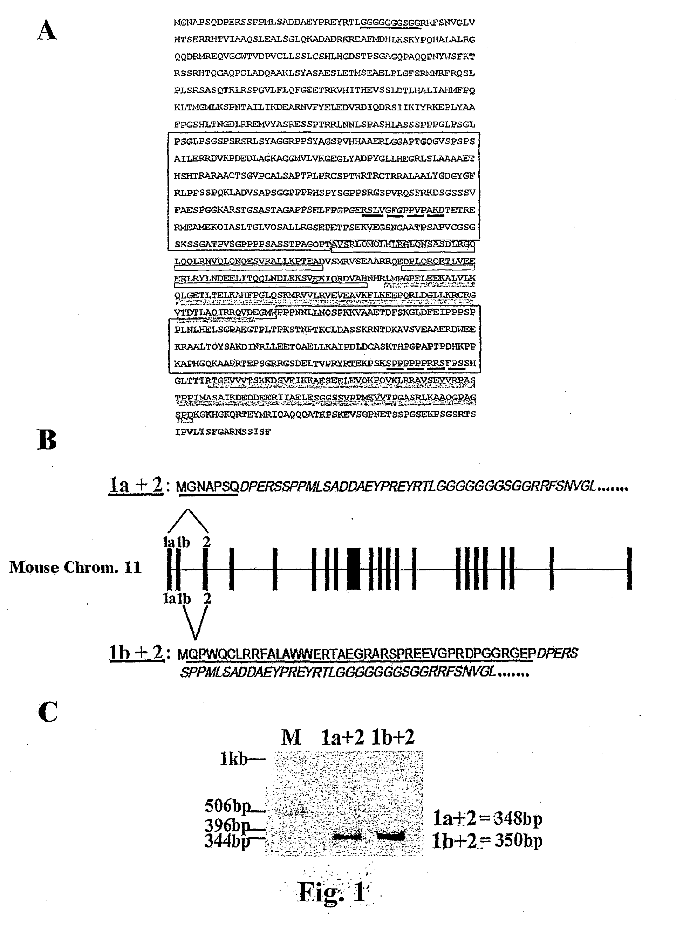

- Figure 1. Genomic organization of p140Cap mouse gene.
   a, p140Cap gene localized on chromosome 11 consists of 24 exons (upper panel). The first two exons, 1a and 1b, are subjected to alternative splicing on exon 2 resulting in mRNA isoforms coding for two proteins which differ in their N-terminal portion (underlined). The sequence of exon 2 is only partially reproduced.
   c, RT-PCR products of the two isoforms of the p140Cap gene was performed on total RNA extracted from mouse brain. cDNA was subjected to PCR using specific oligonucleotides on 1a, 1b and 2 exon sequences. The molecular weights of 1a+2 (348 bp) and 1b+2 (350 bp) amplicons are shown. Note the absence of an amplicon of 651 bp that would have been represented 1a+1b+2. DNA molecular weight ladder is shown on the left. The results are representative of two independent experiments.

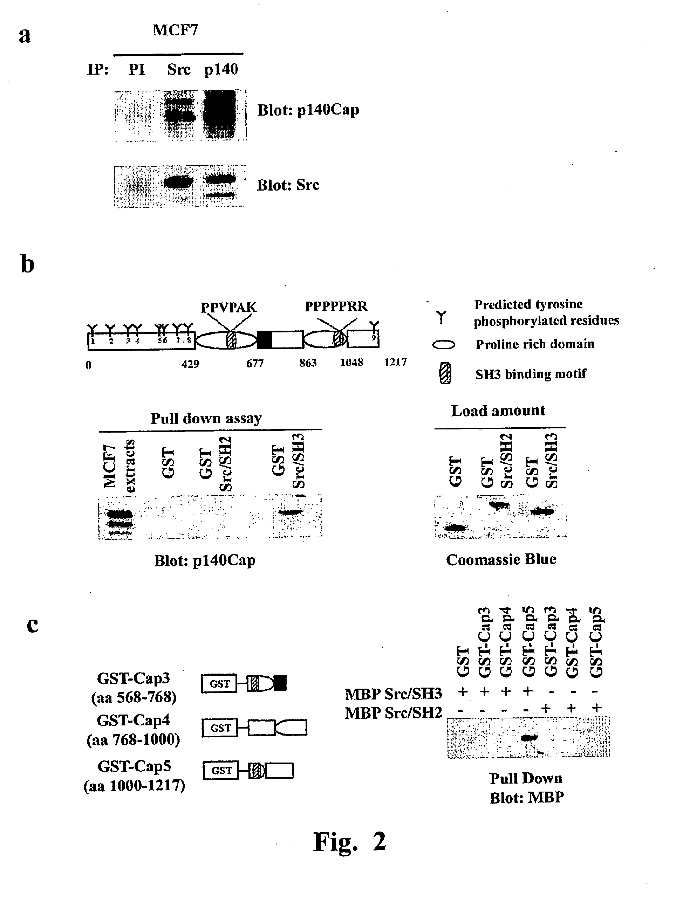

- Figure 2. p140Cap binds directly to Src SH3 domain.
   a, MCF7 cell extracts were immunoprecipitated with monoclonal antibody to p140Cap, Src or pre-immune serum (PI) as negative control. Immunocomplexes were analysed by western blotting using polyclonal antibody to Src and anti p140Cap respectively. The results are representative of six independent experiments.
   b, In the upper panel a schematic representation of full length p140Cap domains is shown. GST, GST-Src/SH2 and GST-Src/SH3 fusion proteins bound to Sepharose columns were used to pull down p140Cap from MCF7 cell extracts. Bound proteins were eluted and analyzed by 8% SDS-PAGE and immunoblotted using p140Cap polyclonal antibody (lower left panel) or stained with Coomassie blu (lower right panel) to quantify the amount of fusion proteins. The results are representative of two independent experiments.
   c, Left panel shows a schematic representation of the different p140Cap GST-fusion proteins used. 2 micrograms of each purified protein was incubated in "in vitro" binding assay with 2 micrograms of MBP-SrcSH2 or MBP-SrcSH3 fusion proteins. Associated proteins were pull-down with Glutathione-Sepharose, eluted and run on 8% SDS-PAGE and immunoblotted with anti MBP antibody (right panel). The results are representative of two independent experiments.

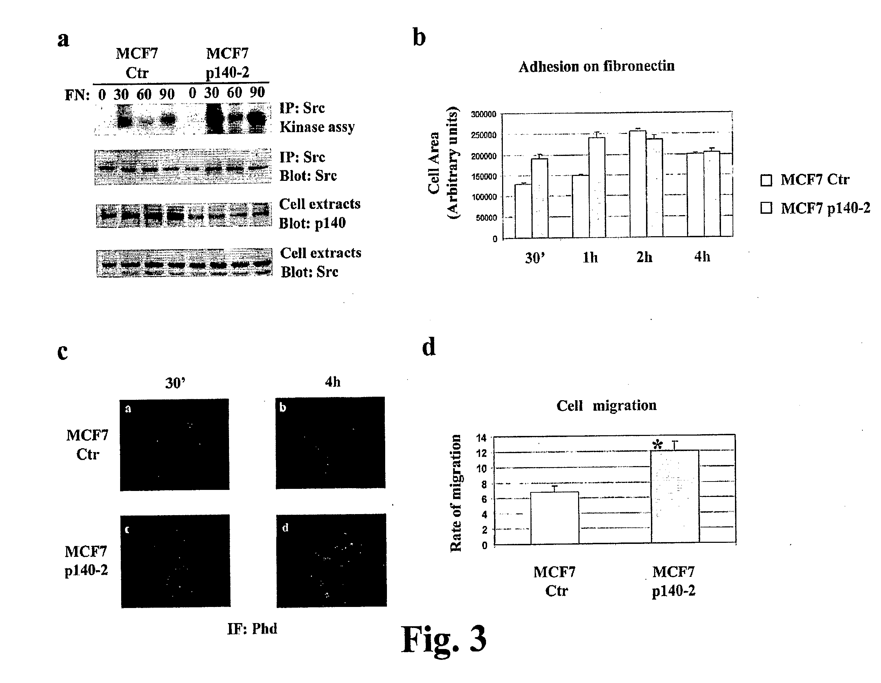

- Figure 3. p140Cap down-regulation by shRNA enhances integrin-dependent Src kinase activity, cells spreading and migration.
   a, MCF7 cells selected for stable expression of p140Cap shRNA (MCF7p140-2) or GFP (MCF7Ctr), were plated on fibronectin (FN) matrix for 30, 60 and 90 minutes or kept in suspension (0). Src was immunoprecipitated and its activity was analysed by in vitro kinase assay. The amount of p140Cap and Src in cell extracts was evaluated by western blotting with specific antibodies.
   b, MCF7Ctr and MCF7p140-2 cells were plated on fibronectin for 30 min, 1, 2 and 4 hrs, fixed and stained with phalloidin (Phd). For each time point, the area of attached cells was calculated by morphometric analysis in 20 random fields (150 cells) at a magnification of 60X, using Metamorph Software. The histogram represents the mean cell area for each time point in arbitrary units. These data are representative of three independent experiments.
   c, MCF7Ctr and MCF7p140-2 cells plated on fibronectin and analysed as in b. Representative fields of three independent experiments are shown at 30 min of adhesion (panels a, c at 100X magnification) and at 4 hours (panels b, d at 40X magnification). Note the fibroblast-like phenotype of MCF7p140-2 cells (panel d) at 4 hours of adhesion.
   d, MCF7Ctr and MCF7p140-2 cells migration was tested in a Transwell assay. 10⁵ cells were seeded on the upper face, let to migrate for 9 hours in the presence or the absence of HGF (25 U/ml), then fixed, stained and counted. Numbers on the y axes represent the fold increase in migration in response to HGF compared to non-stimulated cells. The results are representative of six independent experiments (*p< .05).

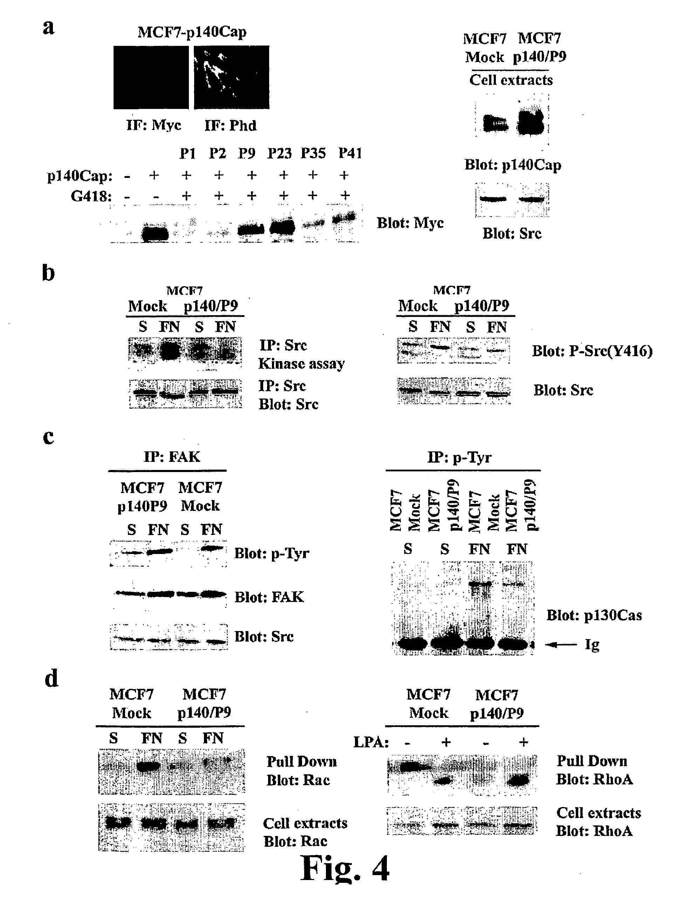

- Figure 4. 140Cap over-expression compromises integrin-dependent Src activation and downstream signalling
   a, MCF7 cells transiently transfected with pcDNA3.1 Myc-p140Cap were plated on glass coverslips and stained for exogenous p140Cap and actin by antibodies to Myc tag and phalloidin (left upper panel). Bar: 20 micrometers. MCF7 cells stable transfected with p140Cap-Myc were analysed by western blotting with anti-Myc tag antibodies (left upper panel). Pool 9 (P9) and pool 23 (P23) expressed similar amount of exogenous p140Cap protein. By western blotting with polyclonal antibodies to p140Cap, P9 cells show a 10 fold increase of total p140Cap expression compared to Mock-transfected cells (right panel). The same blot was reprobed with antibodies to Src kinase.
      MCF7-Mock and MCF7-p140/P9 were plated on fibronectin (FN) or kept in suspension (S) for 30 min.
   b, MCF7-Mock and MCF7-p140/P9, were plated on fibronectin (FN) matrix or kept in suspension (S) for 30 min, extracted and analysed for Src activity by in vitro kinase assay (left panels) or by using a phospho antibody specific for the Src autocatalytic tyrosine 416 (Y416) (right panels). The total amount of Src was evaluated by western blotting with specific antibodies. The results are representative of four independent experiments.
   c, FAK was immunoprecipitated from 1 mg of cell extracts (left panel) and its phsophorylation evaluated by western blotting using a P-Tyr antibody. The amount of immunoprecipitated FAK was quantified using FAK monoclonal antibody. The level of Src co-immunoprecipitated with FAK was detected with monoclonal Src antibody. To detect p130Cas phosphorylation cell extracts were immunoprecipitated with anti phosphotyrosine (P-Tyr) antibodies (right panel) followed by western blotting with a monoclonal antibody to p130Cas.
   d, Activated Rac was pulled down from 1,5 mg of protein extracts with the CRIB domain of PAK and detected by western blotting with monoclonal anti-Rac antibody (left upper panel). Rho activation was induced by treating cells with 1 micromolar LPA for 10 min and analyzed by pull down from 2 mg of protein extracts with the mDIA effector protein. The presence of active Rho A was detected with anti Rho antibody (right upper panel). Total amount of Rac and Rho proteins in cell extracts is shown in the lower panels.

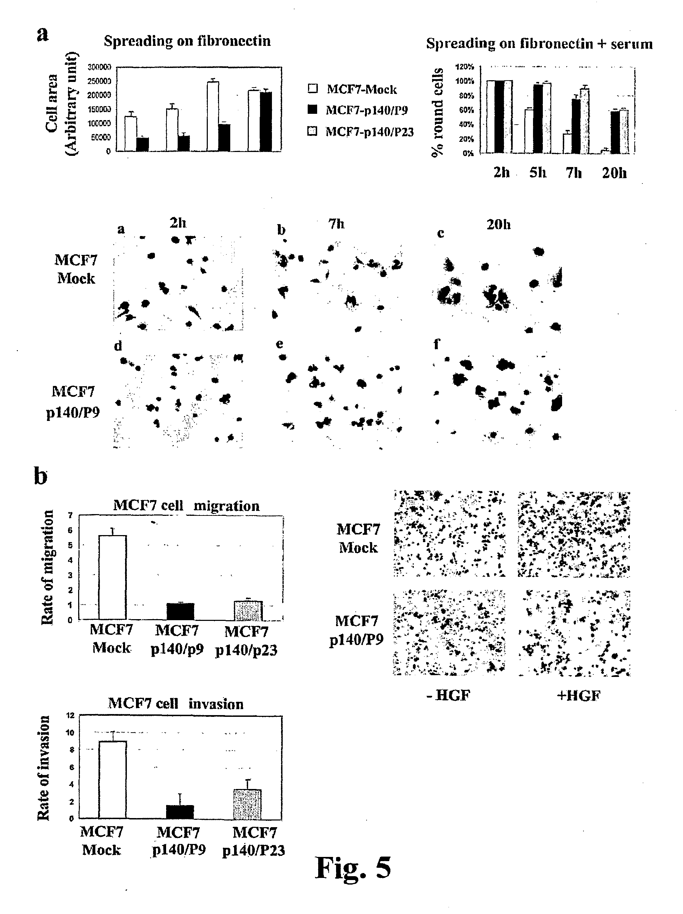

   - Figure 5. p140Cap over-expression inhibits cells spreading, migration and invasion.
   a, Left upper panel, MCF7-Mock and MCF7-p140/P9 cells were plated on fibronectin for 30 min, 1, 2 and 4 hrs, fixed and stained with phalloidin (Phd). For each time point, the area of attached cells was calculated by morphometric analysis in 20 random fields (150 cells) at a magnification of 60X, using Metamorph Software. The histogram represents the mean cell area for each time point in arbitrary units. These data are representative of three independent experiments. Right upper panel, MCF7-Mock and MCF7-p140/P9 and MCF7-p140/P23 cells were plated on fibronectin in the presence of serum for 2, 5, 7 and 20 hours, fixed and stained with Diff Quick kit. The histogram represents the percentage of round cells (surface < 500 micrometer²) over the total number of attached cells (p< .05). The results are representative of four independent experiments.
      Lower panel: MCF7-Mock (a-c) and MCF7-p140/P9 (d-f) cells reported in c, right panel, were photographed at 20X magnification.
   c, MCF7-Mock and MCF7-p140/P9 and MCF7-p140/P23 cells were analysed as shown in Figure 2d in a Transwell assay for 9 hours migration (upper right panel) or 24 hour invasion (lower panel). Numbers on the y axes represent the fold increase in migration in response to HGF compared to non-stimulated cells. The mean values were calculated on six independent experiments (*p< .05). Representative fields (10X magnification) are reported in the upper right panels.

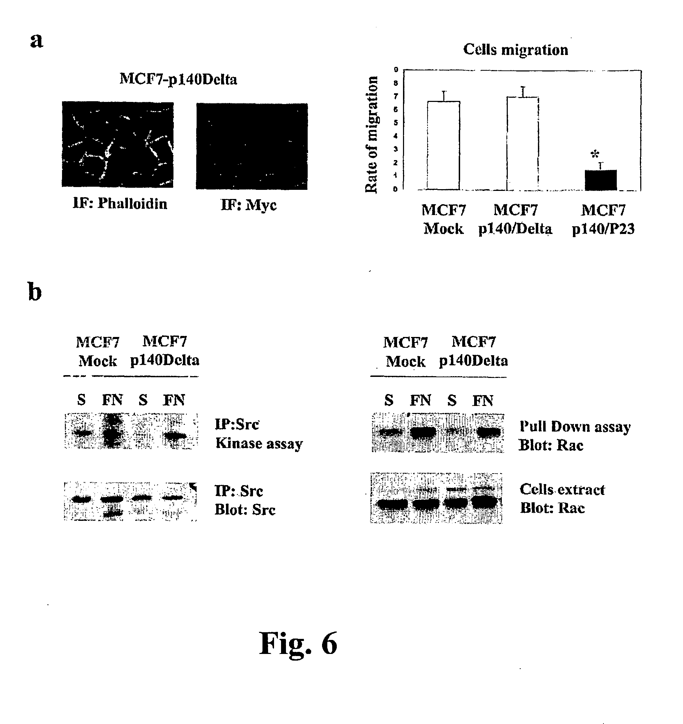

- Figure 6. Src binding domain is essential for p140Cap role in biological processes.
   a, p140Cap and actin localization in MCF7-p140Delta cells, plated on glass coverslips, were analysed by immunofluorescence with monoclonal anti Myc Tag antibody and phalloidin (left panels). MCF7-Mock, MCF7-p140/P23 and MCF7-p140Delta were induced to migrate by HGF stimulus (25U/ml) in a Transwell assay as shown in Figure 2d. Numbers on the y axes of the graphs indicate the increase, in fold, of migration compared to non-stimulated cells (right panel). The results are representative of two independent experiments.
   b, MCF7-Mock and MCF7-p140Delta were plated on fibronectin (FN) for 30 min. Src activity was analyzed by in vitro kinase assay (left panel) as shown in Figure 2a. Activated Rac was pull down from 1.5 mg of protein extracts with the CRIB domain of PAK as shown in Figure 5b. The results are representative of three independent experiments.

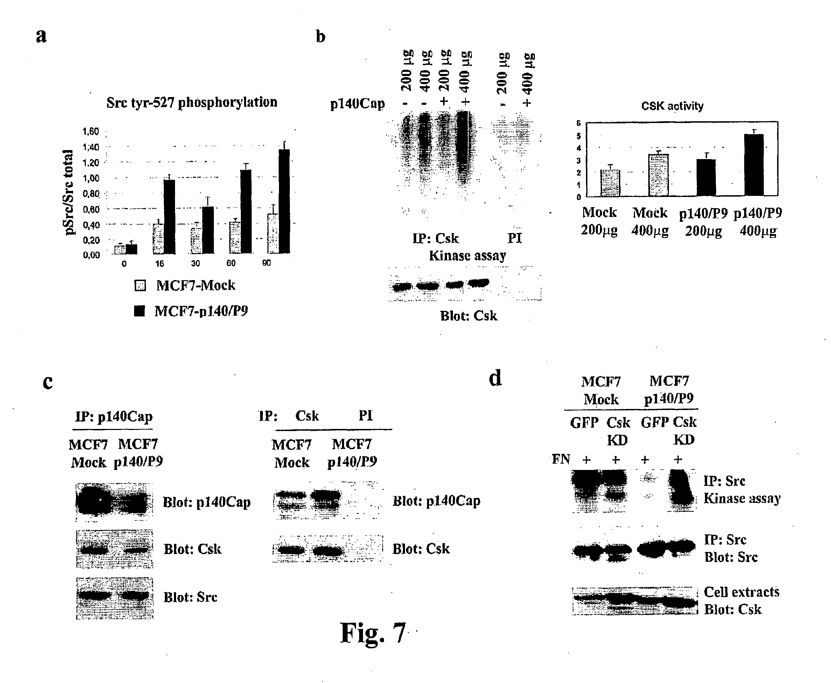

- Figure 7. p140Cap inhibition of Src kinase activity is dependent on Csk activity
   a, MCF7-Mock and MCF7-p140/P9 cells were plated on fibronectin for different times. Phosphorylation of Src Tyr-527 was detected by western blot with specific antibodies. The same blots were reprobed with antibodies to Src. The histogram shows the ratio between Tyr-527 phosphorylation and Src protein level in arbitrary units. This analysis showed a mean 2-2.6 fold increase of Src Tyr-527 phosphorylation in MCF7 - p140/P9 versus MCF7-Mock cells. The results are representative of three independent experiments.
   b, Different amounts (200 microgram or 400microgram) of MCF7-Mock and MCF7-p140/P9 cell extracts were immunoprecipitated with either polyclonal antibody to Csk or preimmune rabbit immunoglobulins (PI). Samples were extensively washed and incubated in a kinase assay using Poly-Glu-Tyr as a tyrosine-kinase-specific substrate (left panel). Densitometric analysis of the phosphorylated Poly-Glu-Tyr signal (right panel) coupled to normalization for Csk protein amounts revealed a 2.3-fold increase of Csk activity in samples derived from MCF7-p140/P9 cells. The results are representative of two independent experiments.
   c, MCF7-Mock and MCF7-p140/P9 cell extracts were immunoprecipitated with monoclonal antibody to p140Cap, Csk or pre-immune serum (PI) as negative control. Immunocomplexes were analysed by western blotting using polyclonal antibody to Src, Csk and p140Cap respectively. The results are representative of six independent experiments.
   d, GFP (GFP) or kinase-negative mutant of Csk (Csk-KD) were introduced into either Mock-MCF7 and MCF7-p140/P9 by recombinant adenovirus infection. Infected cells were plated on fibronectin for 30 minutes. Src activity was analyzed by in vitro kinase assay as shown in Figure 2a. The level of Csk in cell extracts was detected by western blotting using polyclonal antibody to Csk. The results are representative of three independent experiments.

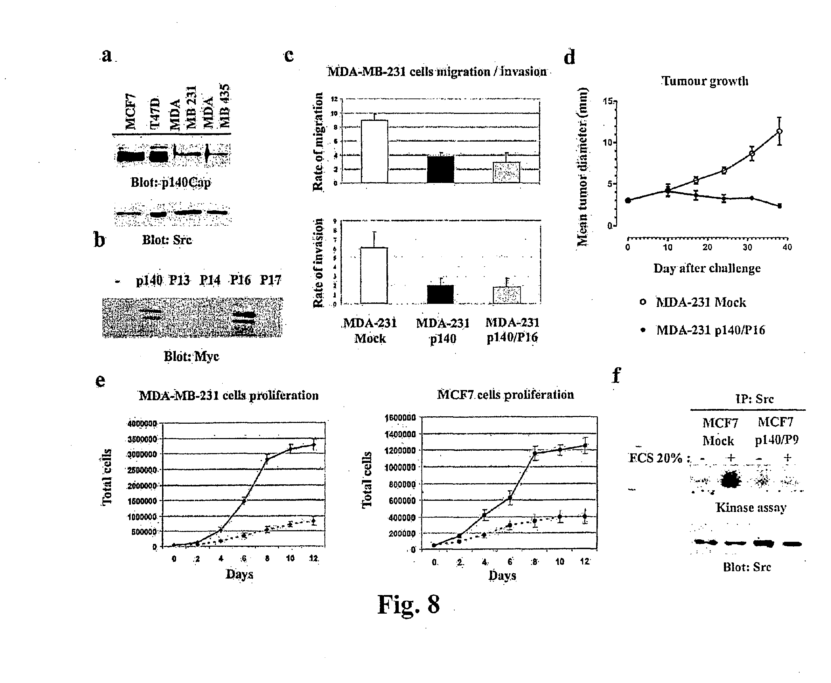

- Figure 8. p140Cap expression correlates with cell migration capacity and in vivo tumour growth
   a, Expression of p140Cap was evaluated in extracts of MCF7, T47D, MDA-MB-231 and MDA-MB-435 breast cancer cells were run by western blot using antibodies specific to p140Cap (upper panel). The blot was reprobed with antibodies specific to Src (lower panel).
   b, MDA-MB-231 cells stable transfected with p140Cap-Myc were analysed by western blotting with anti-Myc tag antibodies. Pool p140 (p140) and pool 16 (P16) were selected for further experiments.
   c, MDA-231 Mock, MDA-231p140 and MDA-231 p140/P16 cells were tested for their ability to migrate and invade using the protocol described in legend to Figure 2. After 2 hours of migration (left panel) and 12 hours of invasion (right panel) cells were fixed, stained and counted. Numbers on the y axes represent the fold increase in migration/invasion in response to HGF compared to non-stimulated cells. The mean values were calculated on five independent experiments (*p< .05).
   d, 10⁷ MDA-231 Mock and MDA-231 p140/P16 cells were injected subcutaneously in SCID mice. Progressively growing neoplastic masses were measured with calipers in two perpendicular diameters and the average value recorded. Differences in tumour volume were evaluated with Student's test.
   e, 10⁵ MDA-231 Mock, MDA-231 p140/P16 (left panel), MCF7-Mock and MCF7-p140/P9 (right panel) cells were plated in 6 cm tissue culture dishes and allowed to grow in the presence of 10% FCS for 12 days. Every two days cells were detached and counted. The mean number of cells from three separate experiments are reported in the Y axis.
   f, MCF7-Mock and MCF7-p140/P9 were starved for 24 hours and treated for 30 min with 20% FCS or left untreated and extracted. Src was immunoprecipitated and its activity was analysed by in vitro kinase assay as described in Experimental procedures. The amount of Src in immunoprecipitates was evaluated by western blotting with specific antibodies. The results are representative of three independent experiments.

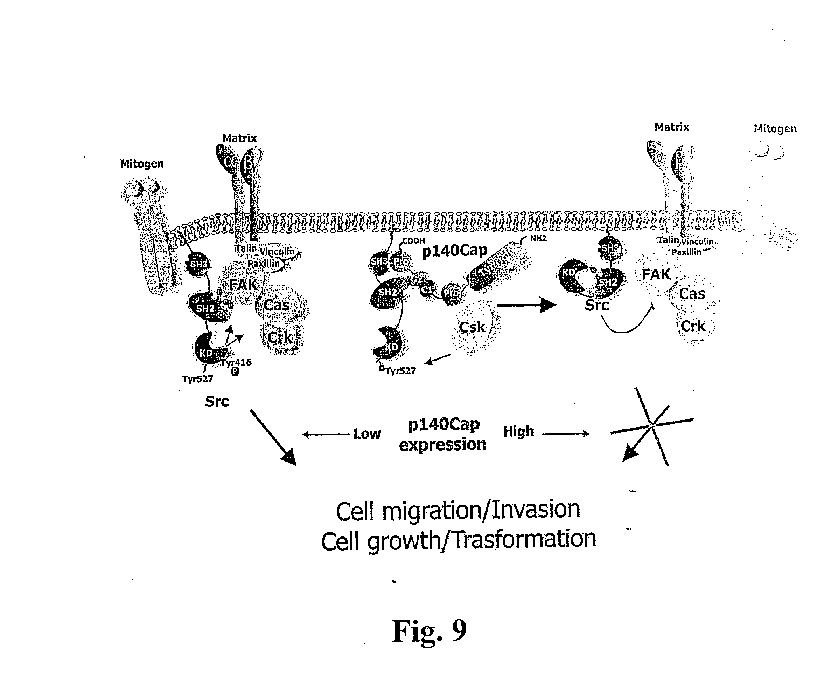

- Figure 9. Schematic summary of the signaling pathways affected by p140Cap.
   Upon cell matrix adhesion or mitogen stimulus, Src is activated and recruits additional transducing molecules such as FAK and p130Cas, leading to cell migration and invasion and regulation of cell growth and transformation. p140Cap participates to these processes by recruiting Src and Csk in a macromolecular complex. High levels of p140Cap expression, by activating Csk, inhibits Src activity and leads to impaired cell spreading, motility, invasion and growth.

### Detailed description of the invention

The present invention will now be described in detail in relation to some preferred embodiments by way of non-limiting examples.

The present invention is directed to the use in the tumor therapy of p140Cap protein and/or fragments thereof, which was surprisingly found able to suppress tumorigenic properties of cancer cells, in particular, breast cancer cells.

In one embodiment, the present invention involves the use of p140Cap protein and/or fragments thereof for the preparation of a medicament for the treatment of tumors and metastases in a patient suffering from a tumor.

p140Cap protein and its fragments are preferably used in pharmaceutical compositions in the form of soluble protein, whose delivery may be achieved using conventional methods of drug delivery. Administration of pharmaceutical compositions comprising p140Cap protein and/or fragments thereof may be accomplished by any method known to the skilled person. For instance, the composition can be administered in an aqueous solution which is injected or infused into the patient. The determination of the proper dosage depends upon a number of case specific variables, including the age and weight of the patient and involve routine experimentation within the expertise of the skilled person .

Another aspect described herein involves the production of a DNA vector comprising a promoter and DNA encoding at least a portion of p140Cap protein. Conservative substitutions in the DNA sequences encoding a portion of p140Cap protein are also encompassed by the present invention, as well as sequences modified by the addition of tag sequences both at the 5' or 3' terminal. Although the production of the DNA vectors of the present invention may be accomplished by a variety of methods known in the art, production is exemplified in Example 11.

The use of a DNA vector comprising DNA encoding at least a portion of p140Cap protein for the preparation of a medicament for the treatment of tumor and metastases in a subject is also described. The delivery of the DNA vectors of the present invention may be achieved using known methods of gene therapy.

Also described is a human p140Cap protein as a target protein for the identification of compounds which may act as agonists of p140Cap protein, and can be useful in the treatment of patients suffering from a tumor, in particular breast tumor.

Also described is a human p140Cap polynucleotide as a target polynucleotide which can be used to identify compounds which may interfere with p140Cap protein expression, in particular over-expression, and can be useful in the treatment of patients suffering from a tumor.

As used herein, the term "compound" is used broadly to mean an organic chemical as a drug; a peptide including a variant or modified peptide-like molecule such as e.g. a peptidomimetic; a protein or fragment thereof; or a polynucleotide molecule. A compound can be a non-naturally occurring compound i.a. produced as a result of *in-vitro* methods or chemical synthesis, or can be a naturally occurring compound such as a protein or fragment thereof expressed i.a. from a cDNA library.

As used herein, the term "expression" means i) the production of an mRNA transcript, or a portion or fragment of an mRNA transcript, of a gene, or ii) the production and/or modification of a protein, or a portion or fragment of a protein, encoded by a gene.

Human p140Cap cDNA (PubMed Accession No. NM_025248; SEQ ID NO.:1) encodes for a protein that is 90% identical to the mouse p140Cap protein isoforms (Figure 1) (cDNA PubMed Accession No. NP_061361, isoform 1a, SEQ ID NO.:2) or locus Q9QWI6 (isoform 1b). Human homology versus rat is 94% identical (PubMed Accession No. XM_001061409). Human homology versus Bos Taurus is 94% (PubMed Accession No. XP_615737).

In this work the present inventors identify the p140Cap as an adaptor protein regulating Src kinase activity and suppressing tumorigenic properties of breast cancer cells. Elevated expression of p140Cap activates the C-terminal Src kinase (Csk) which leads to the inhibition of Src kinase activity, resulting in decreased cell spreading, motility and invasion. Moreover, high levels of p140Cap inhibit cell proliferation and in vivo tumour growth. Accordingly, down-regulation of the endogenous p140Cap protein by RNA interference increases Src kinase activity and drives epithelial breast cancer cells to a more fibroblastic phenotype, leading to enhanced cell migration.

The results presented in the following point-out that p140Cap is a novel Src binding protein. In fact, the p140Cap carboxy-terminal region, characterized by the presence of a proline rich sequence (PPPPPRR, SEQ ID NO.:16), which perfectly matches with the consensus class II PxxPx+ motif known to bind to Src SH3 domains (Kay et al., 2000), interacts in in vitro binding assay with the Src SH3 domain, demonstrating a direct association of these two proteins.

The present data show that Src kinase activity is finely tuned by the levels of expression of p140Cap. In fact, RNA interference experiments demonstrate that a 70% reduction in p140Cap expression is sufficient to up-regulate five fold Src kinase activity over the level of control cells. On the contrary, p140Cap over-expression inhibits Src activation. It is well known that Src kinase activity is negatively controlled by endogenous regulators such as the C-terminal,Src kinase Csk which phosphorylates the inhibitory tyrosine 527 in Src carboxy-terminal domain, allowing the binding of Src SH2 domain and the acquirement of a close, inactive conformation (Gonfloni et al., 2000; Koegl et al., 1995; Yamaguchi and Hendrickson, 1996). The present data show that in p140Cap over-expressing cells, phosphorylation of Src tyrosine 527 is higher than in Mock-transfected cells. Consistent with these data, the present inventors show that p140Cap recruits Csk and Src in a large molecular complex and increases Csk activity leading to down-regulation of Src. Consistently, a Csk kinase-dead mutant rescues Src kinase activity in p140Cap over-expressing cells, demonstrating that p140Cap is a crucial regulator of Src kinase activity through Csk kinase (Fig.8).

In the recent years, many proteins such as the tyrosine kinase FAK have been reported to co-operate with Src in driving downstream signalling either upon cell matrix adhesion or growth factor stimulation (reviewed by (Miranti and Brugge, 2002; Parsons and Parsons, 2004)). The present data show that p140Cap over-expression does not modify FAK phosphorylation, but rather affects its ability to associate with Src, likely because inactive Src does not expose free SH2 domains able to interact with phosphorylated tyrosines on FAK. p140Cap elevated expression also impairs integrin-dependent p130Cas phosphorylation. Formation of a FAK-Src-p130Cas signalling complex has been recently shown to be required for cell invasion through activation of Rac (Hsia et al., 2003). Moreover, cells derived from mice deficient in Src family members (Src/Yes/Fyn) as well as FAK and p130Cas null cells exhibit impaired spreading and significant defects in cell migration (Honda et al., 1999; Ilic et al., 1995; Klinghoffer et al., 1999). According to these results, the present data show that over-expression of p140Cap not only reduces Src/FAK/p130Cas signalling, but also dramatically inhibits cell migration and invasion in breast cancer cell lines with distinct migratory and invasive properties (Lacroix and Leclercq, 2004). These data are further supported by the RNA interference experiments, which, by decreasing the level of p140Cap in MCF7 cells, led to increased motility. Moreover, over-expression of p140Cap impairs spreading on cell matrix and extension of lamellipodia and filopodia, while its down-regulation triggers an increase in cell spreading in the early phases of cell adhesion and induces a fibroblastic-like shape.

Actin cytoskeleton dynamics are controlled by activation of Rho family GTPases (Burridge and Wennerberg, 2004; Etienne-Manneville and Hall, 2002; Jaffe and Hall, 2005). The present data show that elevated levels of p140Cap affects the intracellular balance of Rho GTPases. In particular, elevated levels of p140Cap severely impairs integrin-dependent Rac activity (Price et al., 1998), consistent with the lack of membrane protrusion and of cell migration. On the contrary, p140Cap over-expression up-regulates LPA-dependent RhoA activity, which negatively regulates migratory properties (Arthur and Burridge, 2001). A possible mechanism of RhoA activation might rely on down regulation of p190RhoGAP activity, which in turn depends on active Src (Arthur et al., 2000).

The Src interacting region in the carboxy-terminal domain of p140Cap is required for inhibition of Src and Rac activities and of cell motility. In fact, cells expressing a truncated form of p140Cap lacking the Src binding domain, do not show inhibition of Src and Rac activation as well as do not modify their migration and invasion properties. The observation that the activation of Rac is related to that of Src, is supported by recent data showing that Rac GEFs such as TIAM1 and Vav2 are downstream effectors of Src in epithelial and mesenchimal cells (Servitja et al., 2003).

Taken together these results indicate that p140Cap is a new upstream regulator of Src/FAK/p130Cas signalling in the control of cell motility and invasion (Fig. 8).

Finally, the present data also show that elevated expression of p140Cap in both MCF7 and MDA-MB-231 cells inhibits in vitro and in vivo cell growth, indicating that, in addition to regulate migratory and invasive phenotype, p140Cap also profoundly affects cell proliferation. In standard *in vitro* culture, cells over-expressing p140Cap maintain low degree of confluence, although they do not show obvious signs of cell death (data not shown). The observed defect in cell proliferation is also associated with the loss of Src activation upon serum stimulation, implying that lack of Src activity might be causal to the defective growth. These data also show that p140Cap may down-regulate Src kinase activity not only in response to integrin-mediated adhesion, but also to mitogens and growth factors, indicating that p140Cap might control Src activity upon different stimuli. The defect in proliferation is even more marked in the in vivo model of tumour formation in SCID mice. Mice injected with breast cancer cells expressing high levels of p140Cap do not develop tumours within 40 days, while mice injected with control cells present visible tumours. The mechanisms by which p140Cap over-expression blocks in vivo tumour growth might rely on its ability to activate Csk and to inhibit Src kinase. Although regulation of Src activity by Csk is well defined in *vitro,* no data are available on the role of Csk kinase in breast cancer in vivo. Nevertheless, recent data show that Src activation may promote growth during the process of tumorigenesis (Frame, 2002; Yeatman, 2004). In particular, catalytically inactive Src kinase partially inhibits breast cancer cell tumor formation in nude mice, indicating that Src activity is required for this process (Parsons and Parsons, 2004). However, the absence of tumour formation in mice injected with cells over-expressing p140Cap suggests that additional target of p140Cap, other than Src, might be involved in inhibition of tumour cell growth.

Taken together these results indicate that regulation of p140Cap expression represents a novel way to activate Csk and interfere with Src kinase activity and downstream signalling, affecting the tumorigenic properties of breast cancer cells. Over-expression of p140Cap adaptor might thus constitute a potential tool to revert cancer cells to a more differentiated, less invasive phenotype.

As demonstrated above, p140Cap expression could be down-regulated in human tumours during progression of malignancy, correlating with a negative prognosis of the disease. Accordingly, methods for determining p140Cap expression in human cancer specimens may be employed for diagnostic purposes.

### Reagents and antibodies

Polyclonal and monoclonal antibodies to p140Cap were produced in our laboratory as previously described (Di Stefano et al., 2004). MBP polyclonal antibody was prepared in our laboratory by immunizing rabbit with recombinant MBP protein. Monoclonal and polyclonal antibodies to FAK were purchased by Chemicon (mAb 2156, AB1605). Antibodies to pSrc (pY416 or pY527) and pFAK (pY397) were from Cell Signaling, antibodies PY99 to phosphotyrosine, c-Src, Csk and RhoA were from Santa Cruz Biotechnology. mAb 9E10 to the Myc epitope was purchased from ATCC. MAb 23A8 to Rac was from Upstate biotechnology. Fluoresceine-labelled phalloidin and rhodamine-conjugated anti-rabbit and anti-mouse antibodies were from Sigma. Fluoresceine-conjugated anti-rabbit antibodies were from Jackson Lab. Fibronectin was purified from human plasma as previously described (Defilippi et al., 1994). Human recombinant HGF, cold ATP, Poly Glu-Tyr 4:1 and Coomassie Brilliant Blue were from Sigma. Glutathione-Sepharose, Protein A-Sepharose, Protein G-Sepharose, (gamma-³²P) ATP, nitrocellulose, and films were from Amersham-Pharmacia. Transwell were purchased from Costar (Corning Incorporated). Matrigel matrix basement was from Becton Dickinson. Chemo luminescent ECL reagent was from Euroclone.

### Cell lines, immunofluorescence, immunoprecipitation and western blotting

Tissue culture media, FCS and antibiotics were from Invitrogen. Human breast carcinoma MDA-MB-231, MDA-MB-435, MCF7 and T47D cells, obtained from ATTC, were cultured in RPMI 1640 supplemented with 10% FCS and penicillin/streptomycin (100 U/ml and 100 µg/ml, respectively).

Immunofluorescence was performed as described in (Di Stefano et al., 2004). Briefly, cells were plated on glass coverslips in complete medium for 12 hours. Coverslips were washed in PBS, fixed for 15 minutes with 4% p-formaldehyde, permeabilized with PBS-0.1% Triton X-100, saturated for 30 minutes with 5% goat serum and incubated with primary antibodies (10 microgram/ml) at RT for 60 minutes. After washes in PBS,-0.1% Triton X-100, coverslips were incubated with fluoresceine or rhodamine-labeled secondary antibodies (3 microgram/ml) or phalloidin (2 microgram/ml) for 1 hour, washed and mounted on glass slides with Mowiol. Cell images were visualized on a IX70 Olympus microscope and analyzed with Metamorph software (Universal Imaging Corporation).

For protein analysis, cells were extracted in lysis buffer (60 mM Tris-Cl, 1% NP40, pH 6.8, 0.5% SDS, 1 mM Na₃VO₄, 10 mM NaF, 10 µg /ml leupeptin, 0.4 µg /ml pepstatin and 0.1 U/ml aprotinin). Cell extracts were centrifuged at 13,000×g for 10 minutes, and the supernatants were collected and assayed for protein concentration using the Bio-Rad protein assay method (Bio-Rad).

Proteins were analyzed by SDS-PAGE and western blotting using standard procedures, by detection with peroxidase-conjugated secondary antibodies and chemo luminescent ECL reagent. For immunoprecipitation experiments, pre-cleared cell extracts were incubated for 2 hours at 4°C with 5 microgram/ml purified antibodies or pre-immune sera in the presence of protein-A or protein-G Sepharose beads. The beads were washed three times with 1 ml of TBS 0.5% Triton X-100 and once with 1 ml of TBS 0.5% Triton X-100-0.1% SDS, resolved on SDS-PAGE, transferred onto nitrocellulose and reacted with specific antibodies.

### RNA interference

The shRNA mammalian expression vector (Brummelkamp et al., 2002) pSUPER.retro.puro (OligoEngine) was used. The 140Cap-2 insert specifies an hairpin nucleotide sequence corresponding to nucleotides 2617-2639 downstream of the transcription start site of human p140Cap, separated by a 9-nucleotide non-complementary spacer from the reverse complement of the same 19-nucleotide sequence (SEQ ID NO.:3). This sequence was inserted into the pSUPER.retro.puro backbone after digestion with BgIII and HindIII and the resulting construct was sequenced and referred to as pSuper Retro-p140-2. pSuper Retro-p140-2 and pSuper Retro GFP (negative control) retroviruses were produced by co-transfection of 293GP packaging cells (Invitrogen) with pSR vectors and pVSV-G vector (Clontech). Supernatants were harvested over 48-72 hours, filtrated (0.22 µm pore) and used directly or after concentration (100 X) by ultracentrifugation (50,000 X g for 2hr) as previously described (Piva et al., 2005).

Virus titers were assessed by transducing HeLa cells with serial dilutions of viral stocks. Aliquots of virus, plus 8 µg/ml of polybrene (Sigma), were used to infect exponentially growing MCF7 cells (1x10⁵/ml). Fresh medium was supplemented at 24 hours after the infection. MCF7 cells infected with pSRG retroviruses were enriched by selection with puromycin (1 µg/ml, for 7 days).

### DNA constructs, transfection and analysis of stable over-expressing clones.

Myc-tagged mouse p140Cap full-length cDNA (SEQ ID NO.:2; Di Stefano et al., 2004) and Myc-tagged p140CapDelta deletion mutant prepared by cloning the first 2448 bp of full length cDNA into pcDNA3-Myc plasmid (SEQ ID NO.:4)(PubMed Accession No. NP_061361) were stably transfected in MCF7 or MDA-MB-231 using Lipofectamine 2000 (Invitrogen) following Invitrogen protocols and transiently transfected in HEK293 cells by calcium phosphate precipitation by conventional procedures.

In MCF7 cells, by transfection of (SEQ ID NO.:2) two populations were obtained by selection with 800 microgram/ml of G418 (Gibco) and their expression was determined by 6% SDS-PAGE and western blotting. The populations were named: MCF7-p140/P9 and MCF7-p140/P23. In the same cells by transfection of (SEQ ID NO.:4) one population, named MCF7-p140Delta, was selected. In MDA-MB-231 cells by transfection of (SEQ ID NO.:2) one population was obtained by selection with 800 microgram/ml of G418 (Gibco) and p140Cap expression was determined by 6% SDS-PAGE and western blotting. The population was named MDA-231 p140/P16. Immunofluorescence assay was also used to evaluate the percentage of expressing cells.

### Pull-Down experiments

Constructs encoding the Src GST-tagged fusion proteins containing amino acid residues 146-251(SH2 domain) (SEQ ID NO.:5) and 87-146 (SH3 domain) (SEQ ID NO.:6) in pGEX-2TKX vector were a kind gift of dr. Sarah Courtneidge (Grand Rapids, MI). These fragments were amplified by PCR and cloned into EcoRI-HindIII restriction sites of pMALc2 vector to produce MBP fusion proteins. pGEX cDNA fragments coding for distinct p140Cap regions were described in (Di Stefano et al., 2004).In particular five proteins have been produced as follows:
Cap 1 aminoacids: 1-305 (SEQ ID NO.:7)
Cap 2 aminoacids: 305-568 (SEQ ID NO.:8)
Cap 3 aminoacids: 568-788 (SEQ ID NO.:9)
Cap 4 aminoacids: 788-1000 (SEQ ID NO.:10)
Cap 5 aminoacids: 1000-1217 (SEQ ID NO.:11)

Recombinant GST and MBP fusion proteins were produced in E. Coli and used in pull down assay. Untransfected MCF7 cells were washed in ice-cold PBS and extracted in 750 microliters of GST-fish buffer. Then, 0.5 mg of cell extracts was incubated with 10 microliters of GST-fusion protein beads at 4°C for 1 h. After three washes in GST-fish buffer(10% Glycerol, 50mM Tris pH 7.4, 100mM NaCl, 1%NaP₄O, 2mM MgCl₂, 10 µg/ml each of leupeptin, pepstatin and aprotinin), beads were eluted in 20 microliters of Laemmli sample buffer, and the eluted complexes run on 6% SDS-PAGE and immunoblotted with anti Src mAb (Santa Cruz).
In vitro binding assay to test direct interaction between Src and p140Cap was performed by incubating 2 micrograms of MBP-SrcSH2 or MBP-SrcSH3 with 2 micrograms of each purified p140Cap GST-fusion protein in GST-fish buffer. The complexes were pull down with Glutathione-Sepharose beads for 30 min to 4°C, eluted in Laemmli buffer, run on 8% SDS-PAGE and immunoblotted with anti MBP antibodies.

### Rho GTPases activity assays

For Rac pull-down recombinant GST-PAK CRIB domain fusion protein bound to glutathione-coupled Sepharose 4B beads (Upstate Biotechnology, Lake Placid, NY), was incubated with cell extracts at 4°C for 30 min. The beads were washed three times with an excess of lysis buffer, and bound proteins were eluted in Laemmli buffer and analyzed for the presence of Rac1 molecules by Western blotting using antibodies specific for Rac (Upstate Biotechnology, Lake Placid, NY). Rho activation state was assessed by using GST-mDIA fusion protein to pull down GTP-loaded RhoA accordingly with (Kimura et al., 2000) (Upstate Biotechnology, Lake Placid, NY) from extracts of cells treated with 1 micromolar LPA for 10 min.

### Src and Csk in vitro kinase assay

Cells were washed twice on ice with 1mM Na₃VO₄ and then lysed in 1 ml of lysis buffer. c-Src kinase assay was performed as previously described [44]. Briefly, 1.5 mg of cell extract was pre-cleared using Protein G Sepharose beads and then immunoprecipitated using 5 micrograms of anti-Src mAb for 2-3 hours at 4°C under rotation. Protein G was added at the immunocomplexes for 1 hour, immunocomplexes were then washed four times with RIPA Buffer and then twice with Kinase Buffer (50mM Hepes, pH 7.4, 5mM MgCl₂, 3mM MnCl₂). Immunocomplexes were resuspended in 45 µl of kinase buffer and incubated for 10 minutes at 30 °C with 1nM cold ATP and with 5 microcurie (gamma-³²P) ATP (MIX) in the presence of acid-treated Enolase. Reactions were stopped in Laemli buffer 4X and half were loaded on a 8% SDS-PAGE. The gel was fixed in 10% acetic acid and 10% methanol for 20 minutes, rehydrated in water for 20 minutes, dried and exposed. The other half of the reaction was used to run a parallel gel for immunoblotting and control of equal level of immunoprecipitation.

Csk kinase assay was performed as described for Src kinase assay adding in the reaction 10 micrograms of Poly Glu-Tyr substrate. Reactions were stopped in Laemli buffer 4X and half were loaded on a 7.5% SDS-PAGE. Kinase activity was detected by auto-radiography and calculated by densitometry analisys using Quantity One software (BIO RAD). Recombinant adenoviruses expressing mouse cDNAs for Csk kinase dead mutant (CskKD) (SEQ ID NO.: 13) were used to infect starved MCF7-Mock and MCF7-p140/P9 cells at a multiplicity of infection of 100, followed by incubation for 48 hr in complete medium.

### Cell adhesion, spreading, migration and invasion assay

Cells were serum deprived for 24 hours, detached by 5mM EDTA treatment, washed twice in serum-free DMEM and used for the different assays. For cell adhesion, cells were kept in suspension or plated for different times on 10 microgram/ml fibronectin-coated dishes as previously described in (Moro et al., 2002), extracted and analysed as described above. For cell spreading experiments, cells were plated for different times on 10 microgram/ml fibronectin, washed in PBS, fixed and stained with Diff-Quik kit (Dade Behering).

For migration assays, the lower side of Transwell chambers were coated with 10 microgram/ml fibronectin. 5x10⁴ cells were seeded on the upper side of the filters and incubated in RPMI 0.1% BSA in the presence of 25U/ml HGF (Sigma) in the bottom wells of the chambers. After 2 hours for MDA-MB-231 and 9 hours for MCF7, cells on the upper side of the filters were mechanically removed. Cells migrated to the lower side were fixed and stained with Diff-Quick kit. For invasion assays the upper sides of the filters were coated with 100 microliters of Matrigel matrix basement diluted 1:3 in RPMI. Cells were left to invade for 24 hours for MDA-MB-231 and 48 hours for MCF7 and stained as described above. Cells were counted as described above, analysed with a 20X and 40X objective using Metamorph software.

### Cell proliferation _{'}

10⁵ cells were seeded on 6 cm tissue culture dishes and let to proliferate for twelve days in presence medium supplemented with 10% FCS. Each two days cells were detached and manually counted in Burker chamber on triplicate dishes. The mean number ± SD of counted cells has been reported in a graph.

### In vivo tumor growth

Five-week-old female SCID mice (C.B-17TM/IcrCr1-scidBR) were purchased from Charles River Laboratories (Calco, Italy) and treated in accordance with the European Community guidelines.

The mice were challenged subcutaneously in the left inguinal region with 10⁷ MDA -231 Mock and MDA-231 p140/P16 cells suspended in 200 µl of RPMI and 150 µl Matrigel.

The cages were coded and the incidence and growth of tumors were evaluated twice weekly, with the investigators being blind as to the cell type the animals had received. The two perpendicular diameters of the neoplastic masses were measured with calipers for 40 days. Tumour volume were compared by Student's t test. In the statistical analysis, a p value < 0.05 was considered statistically significant.

### Statistical analysis

The results are representative of at least three independent experiments performed in triplicate and are expressed as the means ± s.e.m. Statistical analysis of the data was performed using a Student's t-test.

### Screening methods

The invention provides assays for screening test compounds which modulate the expression/function of the p140Cap protein.

A test compound preferably binds to p140Cap or mimic p140Cap by binding to effector proteins. More preferably, a test compound increases a biological activity mediated via p140Cap by at least about 10, preferably about 50, more preferably about 75, 90, or 100% relative to the absence of the test compound.

A further test compound preferably regulates expression of p140Cap. More preferably, a test compound up-regulates p140Cap encoding gene by at least about 10, preferably about 50, more preferably about 75, 90, or 100% relative to the absence of the test compound.

### Test Compounds

Test compounds can be pharmacological agents, peptides or proteins already known in the art or can be compounds previously unknown to have any pharmacological activity. Test compounds can be naturally occurring or designed in the laboratory. They can be isolated from microorganisms, animals, or plants, can be produced by recombinant techniques or synthesized by chemical methods known in the art. If desired, test compounds can be obtained using any of the numerous combinatorial library methods known in the art, including but not limited to, biological libraries, spatially addressable parallel solid phase or solution phase libraries, synthetic library methods requiring deconvolution, the "one-bead one-compound" library method, and synthetic library methods using affinity chromatography selection. The biological library approach is limited to polypeptide libraries, while the other four approaches are applicable to polypeptide, non-peptide oligomer, or small molecule libraries of compounds. SeeLam, Anticancer Drug Des. 12, 145,1997.

### Binding assays

For binding assays, the test compound is preferably a small molecule which mimics the ligand binding site of p140Cap, thereby capturing effector molecules such that normal biological activity is prevented. Examples of such small molecules include, but are not limited to, small peptides or peptide-like molecules. Potential effector molecules which bind to a polypeptide of the invention include, but are not limited to, the natural ligands of p140Cap and analogues or derivatives thereof. Natural ligands of p140Cap include but are not limited to: Src family kinases, Csk, SHP-2 PTPase.

In binding assays, the test compound can comprise a detectable label, such as a fluorescent, radioisotopic, chemiluminescent, or enzymatic label, such as horseradish peroxidase, alkaline phosphatase, or luciferase. Detection of a test compound can then be accomplished, for example, by direct counting of radioemission, by scintillation counting, or by determining conversion of an appropriate substrate to a detectable product.

### Functional assays

Test compounds can be tested for the ability to increase a biological effect of a p140Cap polypeptide. Such biological effects can be determined using the functional assays described in the specific examples, below. Functional assays can be carried out after contacting either a purified p140Cap polypeptide, a cell membrane preparation, or an intact cell with a test compound. A test compound which increases a functional activity of a p140Cap by at least about 10, preferably about 50, more preferably about 75,90, or 100% is identified as a potential agent for increasing p140Cap activity.

### p140Cap gene expression

In another embodiment, test compounds which increase p140Cap gene expression are identified. A p140Cap polynucleotide is contacted with a test compound, and the expression of an RNA or polypeptide product of the p140Cap polynucleotide is determined. The level of expression of appropriate mRNA or polypeptide in the presence of the test compound is compared to the level of expression of mRNA or polypeptide in the absence of the test compound. The test compound can then be identified as a modulator of expression based on this comparison. For example, when expression of mRNA or polypeptide is greater in the presence of the test compound than in its absence, the test compound is identified as a stimulator or enhancer of the mRNA or polypeptide expression. Alternatively, when expression of the mRNA or polypeptide is less in the presence of the test compound than in its absence, the test compound is identified as an inhibitor of the mRNA or polypeptide expression.

The level of p140Cap mRNA or polypeptide expression in the cells can be determined by methods well known in the art for detecting mRNA or polypeptide. Either qualitative or quantitative methods can be used. The presence of mRNA and/or polypeptide products of a p140Cap polynucleotide can be determined, for example, using a variety of techniques known in the art, including northern blot, real time PCR, RNA dot blots, quantitative RT-PCR, immunochemical methods such as radioimmunoassay, Western blotting, and immunohistochemistry. Alternatively, polypeptide synthesis can be determined in vivo, in a cell culture, or in an in vitro translation system by detecting incorporation of labeled amino acids into a p140Cap polypeptide.

Such screening can be carried out either in a cell-free assay system or in an intact cell. Any cell which expresses a p140Cap polynucleotide can be used in a cell-based assay system. The p140Cap polynucleotide can be naturally occurring in the cell or can be introduced using techniques such as those described above. Either a primary culture or an established cell line, such as MCF7 or human embryonic kidney 293 cells, can be used.

### Therapeu ti c indications and methods

p140Cap is responsible for many biological functions, and in particular is antagonist to the proliferation of tumor cells. Accordingly, it is desirable to find compounds and drugs which up-regulates the function of p140Cap. For example, compounds which stimulates over-expression of p140Cap and/or activity/functionality may be employed for therapeutic purposes, such as the treatment of tumors.

This specification further pertains to the use of novel agents identified by the screening assays described above. Accordingly, the use a test compound identified as described herein in an appropriate animal model is also described. For example, an agent identified as described herein (e.g., a modulating agent, a specific peptide or protein, etc.) can be used in an animal model to determine the efficacy, toxicity, or side effects of treatment with such an agent.

Furthermore, this specification also describes uses of novel agents identified by the above described screening assays for treatment of tumors and/or metastases in a subject suffering from a tumor. Compounds identified using the screening methods above can be used, for example, for the treatment of breast cancer.

### Examples

### Example 1. p140Cap directly associates to Src kinase.

p140Cap has been identified for its ability to associate with the adaptor protein p130Cas (Di Stefano 2004). As Src kinase is one of the major p130Cas-interacting molecules, it has been evaluated whether Src might also associates to p140Cap. Src and p140Cap were found to reciprocally co-immunoprecipitate in MCF7 breast cancer cells (Figure 2a), indicating that the two molecules are associated in a complex. p140Cap presents two proline-rich regions, potentially involved in binding to SH3 domains, as well as tyrosine residues, which upon phosphorylation, can associate to SH2 domains (Figure 2b, upper panel). GST-fusion proteins expressing SH3 or SH2 Src domains (SEQ ID NO.:6 and 5, respectively) were immobilized on glutathione beads and used to affinity purify endogenous p140Cap from MCF7 cell extracts. Eluted proteins were subjected to western blotting analysis using antibodies against p140Cap. As shown in Figure 2b (lower panel), only the GST-Src SH3 fusion protein pulled down p140Cap, while the GST-Src SH2 recombinant protein was ineffective, indicating that the association between p140Cap and Src is mediated through the Src SH3 domain. To define which of the two p140Cap proline rich regions was required for the binding, different fragments of p140Cap (SEQ ID NO.:7 to 11) were tested in in vitro binding assay with Src SH3 domain. Recombinant GST-Cap 4 and 5 fusion proteins (SEQ ID NO.:10 and 11, respectively), which contain the first and the second proline-rich regions respectively, GST and GST-Cap3 fusion proteins (SEQ ID NO.:9) as negative controls (Figure 2c, left panel) (Di Stefano et al., 2004), were incubated with MBP-SH3 Src fusion protein in an "in vitro" binding assay. As shown in Figure 2c (right panel), only the CapS protein (SEQ ID NO.:11) was able to bind to the MBP-SH3 Src domain, indicating that p140Cap and Src directly interact through the binding of Src SH3 domain with the most carboxy-terminal proline-rich region of p140Cap.

### Example 2. p140Cap down-regulation by shRNA induces integrin-dependent Src kinase activity and enhances cell migration.

To investigate whether the direct association of Src to p140Cap was also affecting Src activity, endogenous p140Cap expression in MCF-7 cells has been down-regulated by RNA interference. MCF7 cells were transiently transfected with six shRNA pSuper Retro (Brummelkamp et al., 2002), covering the entire p140Cap sequence. Among them, the p140-2 (SEQ ID NO.:12) resulted to be the most effective in reducing p140Cap expression (data not shown). Empty pSuper Retro expressing GFP, as negative control (Ctr), and pSuper Retro-p140-2 (p140-2) recombinant retro-viruses were produced in HEK-293 cells and used to infect MCF7 cells to generate stable cell lines. Src kinase activity was analysed in MCF7 Ctr and MCF7 p140-2 cells plated on fibronectin for different times. As shown in Figure 3a, the level of Src activity induced by adhesion to fibronectin was up-regulated by 5 fold at all the examined times in MCF7 p140-2 with a 70% reduction of p140Cap expression. These results indicate that down-regulation of p140Cap lead to enhanced integrin-dependent Src activation.

Src activation in the early phases of cell-matrix adhesion regulates cell spreading and focal adhesion remodelling associated with cell migration and invasion (Yeatman, 2004). Therefore MCF7 p140-2 cells were allowed to adhere and spread on fibronectin for different times and compared to MCF7 Ctr cells. Morphometric analysis showed that within 1 hour of adhesion, MCF7 p140-2 cells presented an increased area (Figure 3b), with extension of large membrane protrusion, thus indicating that down-regulation of p140Cap accelerates cell spreading in the early phases of cell adhesion. At 4 hour of adhesion the difference in the extent of spreading was less evident, however, while MCF7 Ctr cells showed a typical epithelial polygonal morphology, MCF7 p140-2 cells presented a more elongated fibroblastoid phenotype (Figure 3c, panels b and d), as calculated by the mean ratio between cell length and width (MCF7 p140-2 = 3,5; MCF7 Ctr = 2,1). The increased ratio determined in p140-2 expressing cells suggests that down-regulation of p140Cap might mediate a shift to a more fibroblastic phenotype. MCF7 Ctr and MCF7 p140-2 cells were also seeded on fibronectin-coated Transwell filters and allowed to migrate for 9 hours towards 25 U/ml HGF chemoattractant stimulus, a known inducer of cell motility in breast cancer cells. The mean of three experiments (Figure 3d) showed that, upon HGF stimulus, MCF7 p140-2 cells migrated 2 fold more compared to control cells, indicating that down-regulation of p140Cap expression enhances cell migration. Similar results were also obtained by transiently transfecting two siRNA oligos, confirming that the observed effects were not due to retroviral infection (data not shown). Thus stable down-regulation of p140Cap results in increased integrin-dependent Src kinase activity, cell spreading and migratory properties leading to a more fibroblastic phenotype of breast cancer cells.

### Example 3. 140Cap over-expression compromises integrin-dependent Src activation and downstream signalling

The interference experiments demonstrate that p140Cap down-regulation enhances the ability of breast cancer cells to respond to extra cellular matrix in terms of Src activation, cell spreading and increased motility. Therefore it has been tested whether over-expression of p140Cap was sufficient to inhibit integrin-dependent Src activation and downstream signalling. Empty pcDNA3.1 vector or pcDNA3.1-Myc-p140Cap full lenght cDNA (Di Stefano et al., 2004) (SEQ ID NO.:2) were transfected in MCF7 cells and pool of clones were selected. As shown in Figure 4a, two independent pool of clones (MCF7-p140/P9 and MCF7-p140/P23) express ten fold levels of p140Cap compared to MCF7 cells stably transfected with the empty vector (MCF7-Mock) (right panel). Immunofluorescence experiments showed that exogenous p140Cap co-localised with actin structures (Figure 4a, left bottom panel), as the endogenous one (data not shown) indicating that the over-expression of p140Cap did not modify its localisation.

It has been first analysed Src kinase activity in MCF7-Mock and MCF7 over-expressing p140Cap by plating on fibronectin. As shown in Figure 4b (left panel), while in MCF7-Mock cells Src activity was up-regulated within 30 min of adhesion to fibronectin, in MCF7-p140/P9 cells Src activity was not induced. Src kinase activity was also evaluated by phosphospecific antibodies against the critical tyrosine residue 416 in the Src kinase domain. Upon cell-matrix adhesion, tyrosine 416 was phosphorylated only in MCF7-Mock cells, but not in p140Cap over-expressing cells (Figure 4b, right panel). Taken together, these results demonstrate that over-expression of p140Cap results in inhibition of integrin-dependent Src activation.

Upon integrin-mediated adhesion, Src phosphorylates downstream effectors such as FAK and p130Cas (Miranti and Brugge, 2002). To investigate whether p140Cap over-expression affects this signalling, MCF7-Mock as well as MCF7-p140/P9 cells were analyzed for phosphorylation of FAK. As shown in Figure 4c (left panel), p140Cap over-expression did not affect integrin-dependent tyrosine phosphorylation of FAK. However, the amount of Src co-immunoprecipitated with FAK from MCF7-p140/P9 cells was strongly decreased compared to mock-transfected cells. Thus, p140Cap over-expression does not modify integrin-dependent total FAK phosphorylation, but rather its ability to associate to Src. Moreover, integrin-dependent phosphorylation of p130Cas was decreased in cells over-expressing p140Cap (Figure 4c, right panel), indicating that integrin-dependent downstream signalling is affected by p140Cap over-expression, consistent with Src kinase inhibition and reduction of Src/Fak complex.

Rac and RhoA GTPases are known to control actin cytoskeleton organization and cell motility (Burridge and Wennerberg, 2004). To assess whether p140Cap affects their activation, pull down assays to bind and affinity precipitate the activated GTP-bound forms of Rac and RhoA were performed. Interestingly, as shown in Figure 4d, GTP bound Rac was not present in p140Cap over-expressing cells upon adhesion to fibronectin, indicating that p140Cap over-expression inhibits integrin-dependent Rac activation. In the same experimental conditions though, the present inventors were not able to detect any significant activation of RhoA, in accordance with previous reports (Arthur et al., 2000; Degani et al., 2002; Ren et al., 1999). However, LPA treatment strongly activated RhoA in p140Cap over-expressing cells respect to untransfected cells. Taken together all these data indicate that over-expression of p140Cap inhibits integrin-dependent Src activation and its binding to Fak, as well as Rac GTP loading.

### Example 4. p140Cap expression affects cell spreading, migration and invasion of breast cancer cells.

The overall decrease in cell signalling in p140Cap over-expressing cells led the present inventors to investigate the ability of p140Cap transfectants to spread on extracellular matrix and to migrate and invade in Transwell assays. When MCF7-p140/P9 and MCF7-Mock cells were allowed to adhere and spread on fibronectin for different times, morphometric analysis indicated that within 2 hour of adhesion MCF7-p140/P9 cells presented a round morphology and a reduced area (Figure 5a, left panel) compared to control cells, indicating that p140Cap over-expression profoundly inhibits cell spreading ability. Lack of spreading was also observed in the presence of serum: within 20 hours of cell adhesion, while the majority of the MCF7-Mock cells spread on the matrix, 60% of MCF7-p140/P9 and MCF7-p140/P23 cells remained round (Figure 5a, right panel) and the lack of spreading was accompanied by the absence of membrane cell protrusions (Figure 5b). Defective cell spreading was not due to lack of adhesion, since the same number of MCF7-p140/P9 and MCF7-Mock cells adhered to ECM (data not shown). Interestingly both MCF7-p140/P9 and MCF7-Mock cells expressed the same level of beta1 integrin epitope recognised by the mAb 12G10 (Mould et al., 1995), indicating that p140Cap over-expression does not affect beta1 integrin activation state (data not shown). Therefore, 140Cap over-expression severely compromises cell spreading without modifying beta1 integrin activation and cell matrix adhesion.

The inability of MCF7-p140/P9 and MCF7-p140/P23 cells to spread correctly, led the present inventors to investigate the ability of p140Cap transfectants to migrate and invade in Transwell assays. Cells were seeded on fibronectin-coated Transwell filters and allowed to migrate for 9 hours towards 25 U/ml HGF chemoattractant stimulus. As shown in Figure 5c (upper left panel), MCF7-p140/P9 and MCF7-p140/P23 cells were strongly defective in migration in response to the stimuli, while MCF7-Mock cells migrated properly. The mean of six experiments showed that, while, upon HGF stimulus, MCF7-Mock cells migrated 6 fold over untreated cells, cells over-expressing p140Cap only reached 1.5 fold migration rate. Besides cell migration, an "in vitro" invasion assay was performed. MCF7-Mock and MCF7-p140/P9 and MCF7-p140/P23 cells were seeded into Matrigel-coated Transwell and let to invade. Results reported in Figure 5a (lower left panel) show that within 48 hours of invasion, while Mock cells invaded the Matrigel properly in response to the stimulus, MCF7-p140/P9 and MCF7-p140/P23 cells invaded very poorly. Therefore p140Cap over-expression affects the ability of cells to move towards a chemoattractant stimulus and to invade extracellular matrix.

### Example 5. The carboxy-terminal proline-rich region of p140Cap is required for inhibition of c-Src kinase and cell motility and invasion

To assess whether the down-regulation of cell signalling was due to Src binding to p140Cap, MCF7 cells were stably transfected with a cDNA expressing a Myc-tagged truncated form of p140Cap (SEQ ID NO.: 4), lacking the proline-rich Src binding region (MCF7-p140/Delta). By co-immunoprecipitation experiments p140/Delta was shown not to bind with Src (data not shown). By immunofluorescence experiments with anti Myc antibodies, p140/Delta protein was found to localize as the endogenous one (Figure 6a, left panel). In MCF7-p140/Delta cells, "in vitro" Src kinase activity upon fibronectin adhesion was found comparable to that of Mock transfected cells (Figure 6b, left panel), indicating that the Src binding site is required for p140Cap-dependent Src activity inhibition. Similarly, the level of integrin-mediated active Rac was unaffected (Figure 6b, right panel), suggesting that Rac activation is downstream to p140Cap-dependent Src kinase activity. The MCF7-p140/Delta cells were tested for their ability to migrate and invade in Transwell assays. As shown in Figure 6a (right panel), cells expressing the truncated form of p140Cap migrated and invaded at the same rate of control cells. Therefore, these data show that the Src binding site on the p140Cap molecule is essential for down-regulation of downstream signalling and of cell motility and invasion.

### Example 6. p140Cap-dependent Src kinase inhibition is mediated by Csk activation.

C-terminal Src kinase (Csk) is a potent negative regulator of Src due to its ability to phosphorylate the negative regulatory tyrosine 527 on the carboxy-terminal domain of Src (Latour and Veillette, 2001). To investigate its role in p140Cap-dependent inactivation of Src, phosphorylation of tyrosine 527 in MCF7-Mock as well as MCF7-p140/P9 cells was analysed upon adhesion to fibronectin. Phosphospecific antibodies and densitometric analysis showed that the level of tyrosine 527 phosphorylation was increased of 2.5 fold in p140Cap over-expressing cells within 90 min of cell adhesion in comparison to Mock-transfected cells (Figure 7a), suggesting a potential role for p140Cap in regulation of Csk activity. To measure Csk activation, "in vitro" kinase assays were performed on Csk immunoprecipitated using poly-Glu-Tyr as substrate, followed by SDS-PAGE and autoradiography.. As shown in Figure 7b, poly-Glu-Tyr phosphorylation was increased by two fold in p140Cap over-expressing cells compared to Mock transfected cells, demonstrating that p140Cap over-expression up-regulates Csk activity. Reciprocal immunoprecipitation of p140Cap and Csk from wild type MCF7, followed by western blotting with specific antibodies, showed that the two molecules co-immunoprecipitated together with Src (figure 7c), revealing the association of Csk and Src with p140Cap in a macromolecular complex, which could favour the regulation of kinase activities.

To support the role of Csk kinase in p140Cap-dependent inhibition of Src activation, MCF7-Mock and MCF7-p140/P9 cells were infected with adenoviruses expressing a kinase-dead Csk mutant (Csk-KD, PubMed Accession No. CAG46758) and subjected to Src kinase assay upon fibronectin adhesion. As shown in Figure 7d, Src kinase activity was strongly impaired in p140Cap over-expressing cells compared to Mock transfected cells. In contrast, expression of Csk-KD induced a strong activation of Src in MCF7-p140/P9, indicating that the presence of the kinase-dead Csk counteracts the ability of p140Cap to inhibit Src activity. Taken together these data show that p140Cap negatively regulates Src kinase through the activation of Csk kinase.

### Example 7. p140Cap over-expression inhibits tumourigenic properties of aggressive breast cancer cells

To understand the role of p140Cap in breast cancer tumourigenic properties, p140Cap expression was evaluated in different breast cancer cell lines. Based on their phenotype and invasiveness, MCF-7 and T47D or MDA-MB-231 and MDA-MB-435 cells distribute at the boundaries of a spectrum of differentiation from epithelial to mesenchymal phenotype (Lacroix and Leclercq, 2004). Indeed, MCF7 and T47D, which express high amounts of markers typical of the luminal epithelial phenotype of breast cells, are weakly migratory and invasive, while MDA-MB-231 or 435 have a "mesenchymal-like" phenotype and are highly invasive "*in vitro*" and "*in vivo*"*.* As shown in Figure 8a, western blot analysis showed that p140Cap is expressed at low levels in MDA-MB-231 and 435 cell lines compared to MCF7 and T47D cells, suggesting a correlation between levels of p140Cap and morphological and invasive properties of these cells.

To assess this hypothesis pcDNA3.1-Myc-p140Cap full lenght cDNA (SEQ ID NO.: 2) was transfected in highly aggressive breast cancer cells MDA-MB-231 cells and two pool of over-expressing clones were established (MDA-231p140 and MDA-231p140/P16) (Figure 8b) and used for migration and invasion tests in Transwell assays in response to HGF. As shown in Figure 8c, cells over-expressing p140Cap were strongly affected in their capacity to migrate as well as to invade Matrigel compared to Mock cells. Therefore p140Cap over-expression compromises migration and invasion not only in MCF7 cells (see previous paragraphs), but also in an highly invasive breast cancer cell line such as MDA-MB-231.

Tumorigenic properties of breast cancer cells also depend on an high proliferation rate. To assess the role of p140Cap in breast cancer cell growth, 10⁵ MCF7 and MDA-MB-231 cells over-expressing p140Cap (MCF7 p140/P9 and MDA-231p140/P16) and proper controls were seeded in 6 cm dishes and let to grow for 12 days in the presence of FCS. As shown in Figure 8d, cells over-expressing p140Cap were strongly defective in cell proliferation, indicating that high levels of p140Cap decreases the rate of growth. Src kinase activity was also tested in the same experimental conditions: MCF7-Mock and MCF7 over-expressing p140Cap cells were starved and treated with 20% serum. As shown in Figure 7e, while in MCF7-Mock cells Src activity was up-regulated by 30 min of serum treatment, in MCF7-p140/P9 cells Src activity was not induced, thus indicating that p140Cap over-expression inhibits not only integrin-dependent, but also serum-dependent Src activation and impairs the ability of breast cancer cells to grow *in vitro.*

To extend the in vitro data to an in vivo model, 10 x 10⁶ MDA-MB-231 over-expressing p140Cap and proper control were subcutaneously injected in SCID mice. Consistent with the data shown above, 40 days after challenge, while mice injected with MDA-231Mock cells presented a tumour of 1.4 cm mean diameter, mice injected with MDA-231p140/P16 cells were free of tumours (Figure 8f). These data show that cells expressing high levels of p140Cap protein do not grow in in vivo tumorigenic model, suggesting that p140Cap profoundly modify the growth properties of breast cancer cells.

### Example 8. Identification of a test compound that binds to p140Cap protein.

Purified p140Cap protein comprising a glutathione-S-transferase protein and absorbed onto glutathione-derivatized wells of 96-well microtiter plates are contacted with test compounds from a small molecule library at pH 7.0 in a physiological buffer solution. p140Cap protein comprises an amino acid sequence shown in SEQ ID NO.: 1 or 2.

The test compounds comprise a fluorescent tag. The samples are incubated for 5 minutes to one hour. Control samples are incubated in the absence of a test compound.

The buffer solution containing the test compounds is washed from the wells.

Binding of a test compound to P140Cap protein is detected by fluorescence measurements of the contents of the wells. A test compound which increases the fluorescence in a well by at least 15% relative to fluorescence of a well in which a test compound was not incubated is identified as a compound which binds to P140Cap.

### EXAMPLE 9. Isolation of p140Cap-agonist organic compound

In order to identify p140Cap agonists, molecules capable of binding and activating p140Cap function have to be identified. Based on the previously published (DiStefano et al. 2004) and present data, p140Cap binds to Src and affects its activation. Thus, peptides or organic compounds binding to p140Cap and interfering with p140Cap-Src are good candidates as p140Cap agonists by mimicking the p140Cap activity. Such p140Cap agonists can be identified from a large library of peptide and peptide-like compounds by using the techniques of high-throughput screening using a well-defined assay for the detection of such agonists To this end, an ELISA assay can be used in which purified recombinant p140Cap, or fragments of the protein is adsorbed on the surface of microtiter wells are incubated with cell extracts containing Src to allow src-p140Cap binding. To this mixture specific compounds are added to select molecules capable to bind p140Cap. The following ELISA protocol can be used. Fusion proteins consisting of GST (Glutathione S-trasferase) fused to the full-length human p140Cap and/or a fragment from Cap5 protein (SEQ ID NO.: 2 and 11, respectively), containing the Src binding site are purified by affinity chromatography on glutathione-Sepharose 4B. Briefly cells as Human MCF7 or HEK-293 are washed twice with cold PBS and extracted in TBS (25 mMTris-HCl, pH 7.6, 150 mM NaCl, 1 mMNaV0₄, 10 mM NaF, 10 ug/ml leupeptin, 4ug/ml pepstatin and 0.1 TIU/ml aprotinin) 0.5% Nonidet P-40 plus 1 mM Ca²⁺, or with TBS, 0.5% Nonidet P-40 plus 5 mM EDTA to solubilized membrane proteins. Cell extracts containing 2 mg of proteins/ml are incubated overnight at 4 °C in wells coated with GST-p140 fusion proteins. The compounds capable to interfere with p140Cap-src binding will be added at increasing concentrations.

As source of compounds capable to interfere with Src-p140Cap binding, random peptides phage display library can be used (Ladner and Ley 2001). In such libraries random oligonucleotide sequences coding for short amino acid sequences (8-18 residues) are inserted in the coding sequence of the phage coat proteins. The resulting phage displays on its surface the random peptide sequence to be selected for its binding capacity. The peptide sequence coded for by the inserted random oligonucleotide will be determined by DNA sequencing. To allow penetration in the cells, the peptides are coupled to trojan peptides (Derossi et al 1998) that allow spontaneous and efficient intracellular delivery.

Active peptides are also used to develop structural analog organic compounds more suitable for in vivo treatment.

### Example 10. Identification of a test compound which increases p140Cap mRNA expression.

A test compound (p140Cap full length and mutant expressing vectors) is administered by transfection or infection to a culture of human carcinoma cells derived from breast, melanoma, prostate, colon, ovary, uterus, hepatocarcinoma and small cell lung carcinoma and incubated at 37 °C for 2 days. A culture of the same type of cells transfected/infected for the same time without the test compound provides a negative control.

Transfection is performed using a standard procedure with commercially available kits. Infection will be described in Example 11.

RNA is isolated from the two cultures as described in Chozminski and Sacchi. Northern blots are prepared as described in Maniatis and hybridized with ³²P-labeled human P140Cap-specific probe. The probe comprises at least 300 contiguous nucleotides selected from the complement of NM_025248 (human sequence). A test compound which increases the p140Cap-specific signal relative to the signal obtained in the absence of the test compound is identified as an agonist of p140Cap gene expression.

### Example 11. Identification of a test compound which increase P140Cap protein expression.

A test compound (p140Cap full length and mutant expressing vectors) is administered by trasfection/ infection to a culture of human carcinoma cells derived from breast, melanoma, prostate, colon, ovary, uterus, hepatocarcinoma and small cell lung carcinoma and incubated at 37°C for 10 to 45 minutes. A culture of the same type of cells transfected/infected for the same time without the test compound provides a negative control.

Transfection is performed using a standard procedure with commercially available kits. Infection will be described in Example 11.

Cells will be extracted with 1% NP-40 lysis buffer (1% NP-40, 150 mM NaCl, 50 mM Tris-HCl pH 8,5 mM EDTA, 10 mM NaF, 10 mM Na₄P₂O₇, 0.4 mM Na₃VO₄ , 10 µg/ml leupeptin, 4 µg/ml pepstatin and 0.1 unit/ml aprotinin). Cell lysates are centrifuged at 13.000 x g for 10 min and the supernatants are collected and assayed for protein concentration with the Bio-Rad protein assay method. Proteins are run on SDS-PAGE under reducing conditions. Following SDS-PAGE, proteins are transferred to nitrocellulose, incubated with specific antibodies and then detected with peroxidase-conjugate secondary antibodies and chemo luminescent ECL reagent.

### Example 12. Production of viral particles containing sequences for p140Cap expression

As discussed above an alternative therapeutic strategy to prevent and/or cure tumour onset can be achieved by inducing over-expression of p140Cap. Adenovirus constructs and/or other viral vectors such as lentiviral vectors have been proved efficient vector for gene delivery (Wright et al 2001).

Adenoviral vector expressing the p140Cap gene are prepared according to the following protocol given as an example. Lentiviral vectors can be prepared by similar procedures as well. Transfection and viral production is monitored by the fluorescent protein GFP expression. Cells are scraped with a rubber policeman at 7-10 days post-transfection and collected in 50 ml tubes, then spinned in a centrifuge and resuspended in 2 ml sterile PBS. Cells are frozen in dry ice-methanol bath and are thawed in a 37 °C water bath and then vortexed. Cells are frozen and thawed for a total of 4 cycles. Then, samples are spinned briefly and stored at -20 °C. This viral supernatant is used to infect 6650-70% confluent 293 cells in order to produce large amount of viral stocks. The virus are collected when a third to half of the cells are detached. It is possible to confirm the virus presence using PCR and western blot analysis. Viral titer is measured by counting green fluorescent 293 cells 18 hours after infection with various dilutions of virus supernatant. The adenoviral vector is delivered to animals following a catheter-based protocol(Hajjar et al 1998). A viral particle which increases p140Cap expression is administered by infection to a culture of human carcinoma cells derived from breast, melanoma, prostate, colon, ovary, uterus, hepatocarcinoma and small cell lung carcinoma. A culture of the same type of cells infected for the same time with a viral particle that does not interfere with p140Cap expression provides a negative control.

### Example 13. Exemplary Functional assays: cell cycle progression

A test compound which increases p140Cap expression is administered by trasfection/infection to a culture of human carcinoma cells derived from breast, melanoma, prostate, colon, ovary, uterus, hepatocarcinoma and small cell lung carcinoma and incubated at 37°C for 2 days. A culture of the same type of cells transfected/infected for the same time without the test compound provides a negative control.

Actively growing cells are pulsed in a tissue culture flask for one hour with 10 µM BrdU (Sigma, Cat. No. B5002). Cells are detached and poured into a centrifuge tube and centrifuged 10 minutes at 400 x g at RT. Pellet is resuspended by tapping tube and ice cold 70% ethanol to cells is added dropwise, to a final concentration of 1 x 10⁶ cells/100 µl. Incubate 20 minutes at RT, aliquot 100 µl into each test tube (12 mm x 75 mm) and centrifuge 5 minutes. Resuspend pellet in denaturing solution and incubate 20 minutes at RT. Add 1 ml wash buffer. Mix well. Centrifuge 5 minutes. Resuspend pellet in 0.5 ml 0.1 M sodium borate (Na₂B₄O₇), pH 8.5, to neutralize any residual acid. Incubate 2 minutes at RT. Add 1 ml wash buffer. Mix well. Centrifuge 5 minutes. Add primary anti-BrdU monoclonal antibody (Pharmingen) in dilution buffer, Incubate 20 minutes at RT. Add 1 ml wash buffer. Mix well. Centrifuge 5 minutes. Aspirate supernatant. Add secondary antibody: dilute FITC-conjugated goat anti-mouse Ig (PharMingen Cat. No. 12064D) in dilution buffer, such that 50 µl contains the optimal concentration. Resuspend cell pellet in 50 µl of the diluted antibody. Incubate 20 minutes at RT. Add 1 ml wash buffer. Mix well. Centrifuge 5 minutes. Aspirate supernatant. Resuspend pellet in 0.5 ml propidium iodide (10 µg/ml in PBS). Incubate 30 minutes at RT, protected from light. Analyze the cells by flow cytometry, exciting at 488 nm and measuring the BrdU-linked green fluorescence (FITC) through a 514 nm bandpass filter and the DNS linked red fluorescence (PI) through a 600 nm wave-length filter. Following analysis, flush flow cytometer for 10 minutes with 10% bleach and 5 minutes with dH₂O.

### Example 14. Exemplary Functional assays: migration

A test compound which increases p140Cap expression is administered by trasfection/infection to a culture of human carcinoma cells derived from breast, melanoma, prostate, colon, ovary, uterus, hepatocarcinoma and small cell lung carcinoma and incubated at 37°C for 2 days. A culture of the same type of cells transfected/infected for the same time without the test compound provides a negative control.

For migration assays, the lower side of Transwell chambers (Costar) are coated with 10 microgram/ml of fibronectin. 5x10⁴ cells are seeded on the upper side of the filters and incubated in RPMI medium (Gibco) in the presence of 25U/ml HGF (Sigma) in the bottom wells of the chambers. After 2 hours cells on the upper side of the filters are mechanically removed. Cells migrated to the lower side are fixed and stained with Diff-Quick kit and counted.

### Example 15. Exemplary Functional assays: invasion

A test compound which increases p140cap expression is administered by trasfection/infection to a culture of human carcinoma cells derived from breast, melanoma, prostate, colon, ovary, uterus hepatocarcinoma and small cell lung carcinoma and incubated at 37°C for 2 days. A culture of the same type of cells transfected/infected for the same time without the test compound provides a negative control.

For invasion assays the upper of Transwell chambers (Costar) are coated with 100 microliters of Matrigel matrix basement (Becton Dickinson) diluted 1:3 in RPMI medium. 5x10⁴ cells are seeded on the upper side of the filters and let to invade and incubated in RPMI medium (Gibco) in the presence of 25U/ml HGF (Sigma) in the bottom wells of the chambers. Cells were left to invade for 24 or 48 hours and stained with Diff-Quick kit and counted.

### Example 16. Exemplary Functional assays: anchorage-independent growth

A test compound which increases p140Cap expression is administered by trasfection/infection to a culture of human carcinoma cells derived from breast, melanoma, prostate, colon, ovary, uterus, hepatocarcinoma and small cell lung carcinoma and incubated at 37°C for 2 days. A culture of the same type of cells transfected/infected for the same time without the test compound provides a negative control.

For anchorage-independent assays 20 x 10⁴ cells are plated in 6 cm dishes in Basal layer containing 4 ml/dish of 1,2% agar (Difco) in DMEM + antibiotics + 10% FBS with the following procedure. Basal layers are incubated for 16-24 hrs at 37°C in 5% CO₂. 1,5 ml/dish of Top layer consisting of 0,3% agar in DMEM + antibiotics + 10% FBS. Cells are incubated for 17-21 days at 37°C.

### Example 17. Diagnostic method: ELISA

A test compound which increases p140Cap expression is administered by trasfection/infection to a culture of human carcinoma cells derived from breast, melanoma, prostate, colon, ovary, uterus, hepatocarcinoma and small cell lung carcinoma and incubated at 37°C for 2 days. A culture of the same type of cells transfected/infected for the same time without the test compound provides a negative control.

p140Cap concentrations are measured by ELISA The assay format incorporated four different antibodies: (a) goat anti-mouse antibody, (b) mouse anti-p140Cap antibody, (c) rabbit anti-p140cap antibody (d) goat anti-rabbit antibody labeled with biotin. The procedure started with coating of the microtiter plates (96-well Nunc Maxisorb^{™} flat-bottomed plates) with 100 µL/well of goat anti-mouse antibody (3.2 mg/L in 50 mmol/L NaHCO₃/Na₂CO₃, pH 9.6) overnight at 4 °C. The plates were then washed four times with 300 µL/well of washing buffer (1 mL/L Tween 20 in PBS) by use of a 96PW plate washer (SLT Lab Instruments GmbH). The plates were blocked with 10 g/L bovine serum albumin (cat. no. A-7906; Sigma) in PBS (300 µL/well) for 2 h at 37 °C and washed four times with washing buffer. The next step was the incubation with mouse anti-p140Cap antibody antibody [100 µL/well; 0.24 mg/L in dilution buffer (10 g/L bovine serum albumin in washing buffer)] for 2 h at 37 °C. After four washings, the calibrator, the unknown samples, and the reference samples (100 µL/well in dilution buffer) were added to the wells and incubated overnight at 4 °C, after which the plates were again washed four times. All further incubations were performed at ambient temperature. After incubation with rabbit anti-p140Cap antibody (100 µL/well; 0.18 mg/L in dilution buffer), performed for 2 h, the plates were washed and subsequently incubated with 100 µL/well of goat anti-rabbit biotin conjugate (cat. no. B-9642B; Sigma; 2500-fold diluted in dilution buffer) for 2 h and again washed. Thereafter, the plates were incubated with the streptavidin/β-galactosidase conjugate (cat. no. 1112481; Boehringer Mannheim; 2500-fold dilution in dilution buffer; 100 µL/well) for 2 h. The plates were then washed four times and incubated with 100 µL/well of 4-methylumbelliferyl-β-D-galactopyranoside conjugate [cat. no. M-1633; Sigma; 0.075 mmol/L in substrate buffer (100 mmol/L KH₂PO₄/K₂HPO₄ and 1 mmol/L MgCl₂, pH 7.4)] for 1 h. The reaction was stopped by the addition of 100 µL/well of 50 mmol/L NaHCO_{3/}Na₂CO₃ (pH 10.5), and fluorescence was measured with a fluorometric plate reader (Fluoroskan; Lab Systems) using 355 nm excitation and 460 nm emission filters.

### Example 18. Diagnostic method: Immuno Hystochemistry

A human sample from breast, melanoma, prostate, colon, ovary, uterus, hepatocarcinoma and small cell lung carcinoma are fixed using conventional materials ; either formalin or a multi-component fixative, such as FAAG (85% ethanol, 4% formaldehyde, 5% acetic acid, 1% EM grade glutaraldehyde) are adequate for this procedure. Tissue should be fixed at 4°C, either on a sample roller or a rocking platform, for 12 to 48 hours in order to allow fixative to reach the center of the sample. Prior to embedding, samples must be purged of fixative and dehydrated ; this is accomplished through a series of two-to-ten minute washes in increasingly high concentrations of ethanol, beginning at 60%-and ending with two washes in 95%-and another two in 100% ethanol, followed two ten-minute washes in xylene.

Samples can be embedded in a variety of sectioning supports; paraffin, plastic polymers or a mixed paraffin/polymer medium (e. g. ParaplastPlus Tissue Embedding Medium, supplied by Oxford Labware) can be used. For example, fixed, dehydrated tissue can be transferred from the second xylene wash to paraffin or a paraffin/polymer resin in the liquid-phase at about 58°C, then replace three to six times over a period of approximately three hours to dilute out residual xylene, followed by overnight incubation at 58°C under a vacuum, in order to optimize infiltration of the embedding medium in to the tissue. The next day, following several more changes of medium at 20 minute to one hour intervals, also at 58°C, the tissue sample is positioned in a sectioning mold, the mold is surrounded by ice water and the medium is allowed to harden. Sections of 5 um thickness are taken and affixed to 'subbed' slides, which are those coated with a proteinaceous substrate material, usually bovine serum albumin (BSA), to promote adhesion. Other methods of fixation and embedding are also applicable for use according to the methods of the invention; examples of these can be found in Humason, G. L., 1979, Animal Tissue Techniques. 4th ed. (W. H. Freeman & Co., San Francisco).

Primary antibody to p140cap (Monoclonal antibody) is incubated in PBS/Tween-20 at room temperature for 45 minutes to one hour or overnight at 4°C and washed. Visualization of the bound primary antibody complexes can be performed in two ways. The secondary antibody can be complexed either to a fluorescent dye, such as fluorescein or rhodamine, or to an enzyme, such as horseradish peroxidase or alkaline phophatase, that will deposit a colored precipitate when incubated with a solution containing a chromogenic substrate. Both fluorescently-labelled and enzymatically-complexed secondary antibodies are commercially-available from numerous suppliers. Each antibody should be incubated in PBS/Tween-20 at room temperature for 45 minutes to one hour or overnight at 4°C. Again, following incubation with the antibody, slides are washed in PBS/Tween-20 without antibody several times at room temperature, for a total of 1 to 2 hours. If fluorescent dyes are used, bound complexes can be visualized using either a standard fluorescent microscope or, optimally, a confocal microscope. If enzymatic indicators are used, slides should be incubated in the appropriate staining solution according to the manufacturer's literature, and then subjected to direct visual examination.

In either case, a change in intensity of fluorescence or staining of at least five-fold over that observed in neighboring cells or in normal control cells is indicative of abnormal expression of p140Cap. Such a difference may be fifteen-fold, thirty- fold, 100-fold or 500-fold above the level observed in normal cells, and is indicative of malignant cells growth.

### Example 19. Diagnostic method: Tissue Array

A human sample derived from human breast, melanoma, prostate, colon, ovary, uterus, hepatocarcinoma and small cell lung carcinoma is prepared using the Tissue Arrayer Instrument (ATA-100, Chemicon International, Tamecula, California). Four cores are obtained for each tumor in the areas of higher cellularity. Tissue arrays are processed as indicated in example 18.

### Example 20. Diagnostic method: FISH

A human sample from breast, melanoma, prostate, colon, ovary, uterus, hepatocarcinoma and small cell lung carcinoma is subjected to FISH according standard procedures.

The FISH probe containing cDNA for p140Cap labelled with fluorescent dye will be purchased from Qbiogene(MP, formerly ICN)

Slide Preparation: 1. Incubate slide in 0.01M HCl with 0.005% pepsin at 37°C for 10 min. 2. Wash slide 2 x 1 min in PBS at RT. 3. Incubate slide for 10 min in 1% formaldehyde in PBS at RT. 4. Wash slide for 2 x 1 min in PBS at RT. 5. Dehydrate slides in 70%, 95%, 100% Ethanol at RT for 1 min each. 6. Air dry.

Probe Denaturation/Hybridization 1. Aliquot 10 µl of probe for each target into 0.5 ml microfuge tube. 2. Incubate at 96°C for 5 min in water bath: Do not heat probes for more than 5 minutes. 3. Shortly spin the tube in a microcentrifuge. 4. Apply 10 µl of the probe mix to each target and cover with a cover-slip (22x22mm). Care should be taken to avoid air bubbles. 5. Denature slide and probe for 2 min at 80°C on a temperature controlled hot plate. 6. Incubate for 12-18 hours in a humidified environment at 37°C.

Post Hybridization Washing 1. Remove cover-slip by soaking in 2X SSC/0.1% Tween-20 at 37°C. 2. Wash slide 4 x 5 min in 0.5X SSC/0.1% SDS at 60-65°C. 3. Briefly rinse slides in distilled water. 4. Air dry slides out of direct light. 5. Apply. 20 µl DAPI/anti fade solutions to the target and cover with a cover slip. (24x50mm).

### Example 21: Transgenic Mice Generation.

The complete cDNA of mouse p140Cap (SEQ ID NO.: 2) is cloned into the plasmid containing the MMTV promoter, intronic and polyadenylation sequences of simian virus 40, previously cut with Not I and EcoRV. To generate transgenic mice, a fragment corresponding to the insert is purified and microinjected in the pronucleus of fertilized eggs. The injected embryos are transferred to FWB pseudopregnant females and the offspring genotype are tested for the integration of transgene by PCR analysis of genomic DNA. The following oligonucleotides are used for amplification: PM3F CGCAGCACCGGGAGTGCC (SEQ ID No.:14); PM4R CAACTCAGGACCCGGCATTAGC (SEQ ID No.:15). DNA from founder mice that tested positive for PCR is further subjected to Southern analysis to confirm the presence of the transgene. Transgenic lines are generated by mating founder animals to FWB mice and used for analysis of phenotype. Negative littermates serve as a wild-type control. Balb/C Mice MMTV-Neu-T were previously described (Lucchini et al., 1992). p140Cap/Neu-T double transgenic mice were generated by crossing MMTV/p140Cap and MMTV-Neu-T. The inventors first create an outbread FWB/C57 strain and analyzed the first generation by PCR for the presence of the trangenes. The mice that are positive for both the transgenes are included in further analyses.

### Example 22: Morphological and immunohistochemical analysis of transgenic mice

Only mice having litters of at least 8 pups are included in the study. The fourth inguinal glands are used for morphological analysis, cell proliferation and apoptosis assays. For whole-mount staining, mammary glands are flattened on microscopic slides, fixed overnight in Carnoy's solution (75% ethanol, 25% acetic acid) and stained with carmine alum. For histological analysis, mammary glands are fixed overnight in 4% paraformaldehyde in phosphate-buffered saline (PBS), dehydrated in ethanol, cleared in chloroform and embedded in paraffin. Sections of 5 µm are deparaffinized and stained with hematoxylin and eosin.

For Ki67 staining, 5 µm sections are cut and dewaxed, rehydrated and washed in TBS 0.1% Triton X-100 supplemented with NaN₃ to quench peroxidases at room temperature for 10 minutes. The sections are then boiled in a sodium citrate (10 mM) solution in the microwave for 1 minute at high power and for 9 minutes at low power to unmask the antigen. Sections are then saturated with 5 % goat serum in PBS for 20 minutes at room temperature followed by the incubation with 1:50 Ki67 primary antibodies (Novocastra, MIB-1) overnight at 4°C. The day after sections are washed three times 5 minutes each and incubated with PAP anti-mouse 1:200 (Dako) for 30 minutes at room temperature. In the meantime solution A and B (StreptABComplex/HRP, DakoCytomation, Denmark) are prepared according to the manufacturer's instruction, left at 4°C for 30 minutes and then added to the sections once they were washed off the secondary antibodies. The developing solution is incubated on sections for 30 minutes at room temperature and the DAB reaction is followed under the microscope.

### Example 23: Protein analysis and antibodies

Thoracic glands are prepared for Western blot analysis. Briefly, mammary glands are rapidly removed, frozen, pestled in liquid nitrogen, and then boiled in Laemmli Buffer. After a brief sonication, solubilized proteins are centrifuged at 14,000 rpm (4°C) for 5 minutes, and supernatants are stored at -80°C. Protein concentration is evaluated by Biorad DC Protein Assay (Biorad Laboratories). 50 µg of protein extracts are separated by SDS-PAGE and transferred to nitrocellulose. Filters are then blotted with primary antibodies that are obtained from the following sources: p140Cap (Di Stefano et al., 2004), anti-pErk1/2, pAkt, Akt, pGsk3-beta, Gsk3-beta, p-Src, pPTEN, PTEN, (Cell Signaling); Erk1/2, c-Src, Bcl-2, Bcl-xL and pTyr-PY99 (Santa Cruz). Primary antibodies are incubated overnight at 4°C at the dilution suggested by the manufacturer. Peroxidase-conjugated secondary antibodies are purchased from Amersham and used at 1 :10 000. Blots are developed with chemiluminescent system (Euroclone).

Naturally, while the principle of the invention remains the same, the details of construction and the embodiments may widely vary with respect to what has been described and illustrated purely by way of example, without departing from the scope of the present invention as defined in the appended claims.

### References

1. Avizienyte, E., and Frame, M.C. (2005). Src and FAK signalling controls adhesion fate and the epithelial-to-mesenchymal transition. Curr Opin Cell Biol 17, 542-547.
2. Ridley, A.J., Schwartz, M.A., Burridge, K., Firtel, R.A., Ginsberg, M.H., Borisy, G., Parsons, J.T., and Horwitz, A.R. (2003). Cell migration: integrating signals from front to back. Science 302, 1704-1709.
3. Webb, D.J., Donais, K., Whitmore, L.A., Thomas, S.M., Turner, C.E., Parsons, J.T., and Horwitz, A.F. (2004). FAK-Src signalling through paxillin, ERK and MLCK regulates adhesion disassembly. Nat Cell Biol 6, 154-161.
4. Miranti, C.K., and Brugge, J.S. (2002). Sensing the environment: a historical perspective on integrin signal transduction. Nat Cell Biol 4, E83-90.
5. Schwartz, M.A., and Ginsberg, M.H. (2002). Networks and crosstalk: integrin signalling spreads. Nat Cell Biol 4, E65-68.
6. Giancotti, F.G. (2003). A structural view of integrin activation and signaling. Dev Cell 4, 149-151.
7. Cabodi, S.D.P. (2005). The Essence of Integrin Signal Transduction (Springer).
8. Damsky, C.H., and Ilic, D. (2002). Integrin signaling: it's where the action is. Curr Opin Cell Biol 14, 594-602.
9. Frame, M.C., Fincham, V.J., Carragher, N.O., and Wyke, J.A. (2002). v-Src's hold over actin and cell adhesions. Nat Rev Mol Cell Biol 3, 233-245.
10. Burridge, K., and Wennerberg, K. (2004). Rho and Rac take center stage. Cell 116, 167-179.
11. Ishizawar, R., and Parsons, S.J. (2004). c-Src and cooperating partners in human cancer. Cancer Cell 6, 209-214.
12. Yeatman, T.J. (2004). A renaissance for SRC. Nat Rev Cancer 4, 470-480.
13. Matsumoto, T., Jiang, J., Kiguchi, K., Ruffino, L., Carbajal, S., Beltran, L., Bol, D.K., Rosenberg, M.P., and DiGiovanni, J. (2003). Targeted expression of c-Src in epidermal basal cells leads to enhanced skin tumor promotion, malignant progression, and metastasis. Cancer Res 63, 4819-4828.
14. Irby, R.B., and Yeatman, T.J. (2000). Role of Src expression and activation in human cancer. Oncogene 19, 5636-5642.
15. Biscardi, J.S., Ishizawar, R.C., Silva, C.M., and Parsons, S.J. (2000). Tyrosine kinase signalling in breast cancer: epidermal growth factor receptor and c-Src interactions in breast cancer. Breast Cancer Res 2, 203-210.
16. Boyer, B., Roche, S., Denoyelle, M., and Thiery, J.P. (1997). Src and Ras are involved in separate pathways in epithelial cell scattering. Embo J 16, 5904-5913.
17. Ilic, D., Furuta, Y., Kanazawa, S., Takeda, N., Sobue, K., Nakatsuji, N., Nomura, S., Fujimoto, J., Okada, M., and Yamamoto, T. (1995). Reduced cell motility and enhanced focal adhesion contact formation in cells from FAK-deficient mice. Nature 377, 539-544.
18. Klinghoffer, R.A., Sachsenmaier, C., Cooper, J.A., and Soriano, P. (1999). Src family kinases are required for integrin but not PDGFR signal transduction. Embo J 18, 2459-2471.
19. Mitra, S.K., Hanson, D.A., and Schlaepfer, D.D. (2005). Focal adhesion kinase: in command and control of cell motility. Nat Rev Mol Cell Biol 6, 56-68.
20. Shin, N.Y., Dise, R.S., Schneider-Mergener, J., Ritchie, M.D., Kilkenny, D.M., and Hanks, S.K. (2004). Subsets of the Major Tyrosine Phosphorylation Sites in Crk-associated Substrate (CAS) Are Sufficient to Promote Cell Migration. J Biol Chem 279, 38331-38337.
21. Goldberg, G.S., Alexander, D.B., Pellicena, P., Zhang, Z.Y., Tsuda, H., and Miller, W.T. (2003). Src phosphorylates Cas on tyrosine 253 to promote migration of transformed cells. J Biol Chem 278, 46533-46540.
22. Chodniewicz, D., and Klemke, R.L. (2004). Regulation of integrin-mediated cellular responses through assembly of a CAS/Crk scaffold. Biochim Biophys Acta 1692, 63-76.
23. Ilic, D., Almeida, E.A., Schlaepfer, D.D., Dazin, P., Aizawa, S., and Damsky, C.H. (1998). Extracellular matrix survival signals transduced by focal adhesion kinase suppress p53-mediated apoptosis. J Cell Biol 143, 547-560.
24. Di Stefano, P., Cabodi, S., Boeri Erba, E., Margaria, V., Bergatto, E., Giuffrida, M.G., Silengo, L., Tarone, G., Turco, E., and Defilippi, P. (2004). P130Cas-associated protein (p140Cap) as a new tyrosine-phosphorylated protein involved in cell spreading. Mol Biol Cell 15, 787-800.
25. Brummelkamp, T.R., Bernards, R., and Agami, R. (2002). A system for stable expression of short interfering RNAs in mammalian cells. Science 296, 550-553.
26. Ren, X.D., Kiosses, W.B., and Schwartz, M.A. (1999). Regulation of the small GTP-binding protein Rho by cell adhesion and the cytoskeleton. Embo J 18, 578-585.
27. Arthur, W.T., Petch, L.A., and Burridge, K. (2000). Integrin engagement suppresses RhoA activity via a c-Src-dependent mechanism. Curr Biol 10, 719-722.
28. Degani, S., Balzac, F., Brancaccio, M., Guazzone, S., Retta, S.F., Silengo, L., Eva, A., and Tarone, G. (2002). The integrin cytoplasmic domain-associated protein ICAP-1 binds and regulates Rho family GTPases during cell spreading. J Cell Biol 156, 377-387.
29. Mould, A.P., Garratt, A.N., Askari, J.A., Akiyama, S.K., and Humphries, M.J. (1995). Identification of a novel anti-integrin monoclonal antibody that recognises a ligand-induced binding site epitope on the beta 1 subunit. FEBS Lett 363, 118-122.
30. Latour, S., and Veillette, A. (2001). Proximal protein tyrosine kinases in immunoreceptor signaling. Curr Opin Immunol 13, 299-306.
31. Lacroix, M., and Leclercq, G. (2004). Relevance of breast cancer cell lines as models for breast tumours: an update. Breast Cancer Res Treat 83, 249-289.
32. Kay, B.K., Williamson, M.P., and Sudol, M. (2000). The importance of being proline: the interaction of proline-rich motifs in signaling proteins with their cognate domains. Faseb J 14, 231-241.
33. Gonfloni, S., Weijland, A., Kretzschmar, J., and Superti-Furga, G. (2000). Crosstalk between the catalytic and regulatory domains allows bidirectional regulation of Src. Nat Struct Biol 7, 281-286.
34. Koegl, M., Courtneidge, S.A., and Superti-Furga, G. (1995). Structural requirements for the efficient regulation of the Src protein tyrosine kinase by Csk. Oncogene 11, 2317-2329.
35. Yamaguchi, H., and Hendrickson, W.A. (1996). Structural basis for activation of human lymphocyte kinase Lck upon tyrosine phosphorylation. Nature 384, 484-489.
36. Parsons, S.J., and Parsons, J.T. (2004). Src family kinases, key regulators of signal transduction. Oncogene 23, 7906-7909.
37. Hsia, D.A., Mitra, S.K., Hauck, C.R., Streblow, D.N., Nelson, J.A., Ilic, D., Huang, S., Li, E., Nemerow, G.R., Leng, J., Spencer, K.S., Cheresh, D.A., and Schlaepfer, D.D. (2003). Differential regulation of cell motility and invasion by FAK. J Cell Biol 160, 753-767.
38. Honda, H., Nakamoto, T., Sakai, R., and Hirai, H. (1999). p130(Cas), an assembling molecule of actin filaments, promotes cell movement, cell migration, and cell spreading in fibroblasts. Biochem Biophys Res Commun 262, 25-30.
39. Etienne-Manneville, S., and Hall, A. (2002). Rho GTPases in cell biology. Nature 420, 629-635.
40. Jaffe, A.B., and Hall, A. (2005). RHO GTPases: Biochemistry and Biology. Annu Rev Cell Dev Biol.
41. Price, L.S., Leng, J., Schwartz, M.A., and Bokoch, G.M. (1998). Activation of Rac and Cdc42 by integrins mediates cell spreading. Mol Biol Cell 9, 1863-1871.
42. Arthur, W.T., and Burridge, K. (2001). RhoA inactivation by p190RhoGAP regulates cell spreading and migration by promoting membrane protrusion and polarity. Mol Biol Cell 12, 2711-2720.
43. Servitja, J.M., Marinissen, M.J., Sodhi, A., Bustelo, X.R., and Gutkind, J.S. (2003). Rac1 function is required for Src-induced transformation. Evidence of a role for Tiam1 and Vav2 in Rac activation by Src. J Biol Chem 278, 34339-34346.
44. Frame, M.C. (2002). Src in cancer: deregulation and consequences for cell behaviour. Biochim Biophys Acta 1602, 114-130.
45. Cabodi, S., Moro, L., Baj, G., Smeriglio, M., Di Stefano, P., Gippone, S., Surico, N., Silengo, L., Turco, E., Tarone, G., and Defilippi, P. (2004). p130Cas interacts with estrogen receptor alpha and modulates non-genomic estrogen signaling in breast cancer cells. J Cell Sci 117, 1603-1611.
46. Defilippi, P., Bozzo, C., Volpe, G., Romano, G., Venturino, M., Silengo, L., and Tarone, G. (1994). Integrin-mediated signal transduction in human endothelial cells: analysis of tyrosine phosphorylation events. Cell Adhes Commun 2, 75-86.
47. Kimura, K., Tsuji, T., Takada, Y., Miki, T., and Narumiya, S. (2000). Accumulation of GTP-bound RhoA during cytokinesis and a critical role of ECT2 in this accumulation. J Biol Chem 275, 17233-17236.
48. Moro, L., Dolce, L., Cabodi, S., Bergatto, E., Erba, E.B., Smeriglio, M., Turco, E., Retta, S.F., Giuffrida, M.G., Venturino, M., Godovac-Zimmermann, J., Conti, A., Schaefer, E., Beguinot, L., Tacchetti, C., Gaggini, P., Silengo, L., Tarone, G., and Defilippi, P. (2002). Integrin-induced epidermal growth factor (EGF) receptor activation requires c-Src and p130Cas and leads to phosphorylation of specific EGF receptor tyrosines. J Biol Chem 277, 9405-9414.

## Claims

1. p140Cap protein, and/or fragments thereof for use in the treatment of tumor and/or metastases in a patient suffering from a tumor.

2. p140Cap protein, and/or fragments thereof for use according to claim 1, **characterized in that** said protein, and/or fragments thereof are in a soluble form.

3. p140Cap protein, and/or fragments thereof for use according to claim 1, **characterized in that** said p140Cap protein, and/or fragments thereof is administered by injection or by infusion.

4. Portion of the nucleotide sequence encoding p140Cap protein for use in the treatment of tumor and/or metastases in a patient suffering from tumor.

5. Vector comprising at least a portion of the nucleotide sequence encoding p140Cap protein, for use in the treatment of tumor and/or metastases in a subject suffering from tumor, wherein said vector is a viral vector, and wherein said vector expresses at least a portion of the nucleotide sequence encoding p140Cap protein in the tumor.

6. Vector for use according to claim 5, **characterized in that** said vector is in the form of a particle.

7. Use of a polynucleotide encoding at least a portion of p140Cap protein or a polypeptide comprising at least a portion of p140Cap protein for screening for pharmacologically active compounds useful in the treatment of tumors.

8. Use according to claim 7, **characterized in that** said pharmacologically active compound is a p140Cap agonist.

9. Use according to claim 7, **characterized in that** said pharmacologically active compound interferes with the expression of p140Cap protein, preferably said pharmacologically active compound determines over-expressing p140Cap protein.

10. Use according to any one of claims 1, 5 to 9, wherein the tumor is a solid tumor, preferably a breast, stomach, colon, prostate, uterus, ovary, pancreas tumor.

11. Method for diagnosis or prognosis of abnormal cell growth, the method comprising measuring the expression of p140Cap gene in a biological sample from a patient.

12. Method according to claim 11, **characterized in that** said method is an immunochemical method, a DNA hybridization method or an RNA hybridization method.

13. Method according to claim 11 or claim 12, **characterized in that** said abnormal cell growth is a malignant cell growth.

14. Non-human mammalian transgenic animal, being transgenic for having altered p140Cap and Neu-T expression, wherein said altered p140Cap expression is performed by stable modification of p140Cap expression at transcriptional, translational or post-translational level, wherein said altered p140Cap expression is an over-activation of p140Cap gene and wherein said non-human mammalian transgenic animal having altered p140Cap and Neu-T expression is generated by crossing a p140Cap transgenic animal with a Neu-T transgenic animal.

15. Non-human mammalian transgenic animal according to claim 14, wherein said non-human mammalian transgenic animal belongs to the murine genus (mus musculus).

16. Cells derivable from the non-human mammalian transgenic animal according to any of claims 14 to 15 and having altered p140Cap and Neu-T expression.

17. Cells according to claim 16, **characterized in that** said cells carry a mutation over-activating p140Cap gene.

## Patentansprüche

1. p140Cap-Protein und/oder Fragmente davon zur Verwendung bei der Behandlung eines Tumors und/oder von Metastasen bei einem Patienten, der an einem Tumor leidet.

2. p140Cap-Protein und/oder Fragmente davon zur Verwendung gemäß Anspruch 1, **dadurch gekennzeichnet, dass** das Protein und/oder die Fragmente davon in einer löslichen Form vorliegen.

3. p140Cap-Protein und/oder Fragmente davon zur Verwendung gemäß Anspruch 1, **dadurch gekennzeichnet, dass** das p140Cap-Protein und/oder Fragmente davon durch Injektion oder durch Infusion verabreicht wird.

4. Teil der p140Cap-Protein kodierenden Nucleotidsequenz zur Verwendung bei der Behandlung eines Tumors und/oder von Metastasen bei einem Patienten, der an einem Tumor leidet.

5. Vektor, umfassend mindestens einen Teil der p140Cap-Protein kodierenden Nucleotidsequenz, zur Verwendung bei der Behandlung eines Tumors und/oder von Metastasen bei einem Probanden, der an einem Tumor leidet, wobei es sich bei dem Vektor um einen Virusvektor handelt und wobei der Vektor mindestens einen Teil der p140Cap-Protein kodierenden Nucleotidsequenz in dem Tumor exprimiert.

6. Vektor zur Verwendung gemäß Anspruch 5, **dadurch gekennzeichnet, dass** der Vektor in der Form eines Partikels vorliegt.

7. Verwendung eines Polynucleotids, das mindestens einen Teil des p140Cap-Proteins kodiert, oder eines Polypeptids, das mindestens einen Teil des p140Cap-Proteins umfasst, zum Screening auf pharmakologisch wirksame, bei der Behandlung von Tumoren nützliche Verbindungen.

8. Verwendung gemäß Anspruch 7, **dadurch gekennzeichnet, dass** es sich bei der pharmakologisch wirksamen Verbindung um einen p140Cap-Agonisten handelt.

9. Verwendung gemäß Anspruch 7, **dadurch gekennzeichnet, dass** die pharmakologisch wirksame Verbindung die Expression des p140Cap-Proteins stört, vorzugsweise bestimmt die pharmakologisch wirksame Verbindung das Überexprimieren von p140Cap-Protein.

10. Verwendung gemäß einem der Ansprüche 1, 5 bis 9, wobei es sich bei dem Tumor um einen soliden Tumor, vorzugsweise einen Brust-, Magen-, Dickdarm-, Prostata-, Uterus-, Eierstock-, Pankreastumor, handelt.

11. Verfahren zur Diagnose oder Prognose von abnormalem Zellwachstum, wobei das Verfahren umfasst, die Expression des p140Cap-Gens in einer biologischen Probe von einem Patienten zu messen.

12. Verfahren gemäß Anspruch 11, **dadurch gekennzeichnet, dass** es sich bei dem Verfahren um ein immunchemisches Verfahren, ein DNA-Hybridisierungsverfahren oder ein RNA-Hybridisierungsverfahren handelt.

13. Verfahren gemäß Anspruch 11 oder Anspruch 12, **dadurch gekennzeichnet, dass** es sich bei dem abnormalen Zellwachstum um ein malignes Zellwachstum handelt.

14. Nicht menschliches transgenes Säugetier, das insofern transgen ist, als es veränderte p140Cap- und Neu-T-Expression aufweist, wobei die veränderte p140Cap-Expression durch stabile Modifikation der p140Cap-Expression auf transkriptionaler, translationaler oder posttranslationaler Ebene durchgeführt wird, wobei es sich bei der veränderten p140Cap-Expression um eine Überaktivierung des p140Cap-Gens handelt und wobei das nicht menschliche transgene Säugetier, das veränderte p140Cap- und Neu-T-Expression aufweist, durch Kreuzen eines für p140Cap transgenen Tiers mit einem für Neu-T transgenen Tier erzeugt wird.

15. Nicht menschliches transgenes Säugetier gemäß Anspruch 14, wobei das nicht menschliche transgene Säugetier zur Mäusegattung (Mus musculus) gehört.

16. Zellen, die von dem nicht menschlichen transgenen Säugetier gemäß einem der Ansprüche 14 bis 15 abgeleitet werden können und veränderte p140Cap- und Neu-T-Expression aufweisen.

17. Zellen gemäß Anspruch 16, **dadurch gekennzeichnet, dass** die Zellen eine Mutation tragen, die das p140Cap-Gen überaktiviert.

## Revendications

1. Protéine p140Cap et/ou fragments de celle-ci destinés à être utilisés dans le traitement d'une tumeur et/ou de métastases chez un patient souffrant d'une tumeur.

2. Protéine p140Cap et/ou fragments de celle-ci destinés à être utilisés selon la revendication 1, **caractérisés en ce que** ladite protéine et/ou fragments de celle-ci sont sous une forme soluble.

3. Protéine p140Cap et/ou fragments de celle-ci destinés à être utilisés selon la revendication 1, **caractérisés en ce que** ladite protéine p140Cap et/ou fragments de celle-ci sont administrés par une injection ou par perfusion.

4. Partie de la séquence de nucléotides codant pour la protéine p140Cap destinée à être utilisée dans le traitement d'une tumeur et/ou de métastases chez un patient souffrant d'une tumeur.

5. Vecteur comprenant au moins une partie de la séquence de nucléotides codant pour la protéine p140Cap, destiné à être utilisé dans le traitement d'une tumeur et/ou de métastases chez un sujet souffrant d'une tumeur, où ledit vecteur est un vecteur viral, et où ledit vecteur exprime au moins une partie de la séquence de nucléotides codant pour la protéine p140Cap dans la tumeur.

6. Vecteur destiné à être utilisé selon la revendication 5, **caractérisé en ce que** ledit vecteur est sous la forme d'une particule.

7. Utilisation d'un polynucléotide codant pour au moins une partie de la protéine p140Cap ou d'un polypeptide comprenant au moins une partie de la protéine p140Cap afin de cribler des composés pharmacologiquement actifs utiles dans le traitement de tumeurs.

8. Utilisation selon la revendication 7, **caractérisée en ce que** ledit composé pharmacologiquement actif est un agoniste de p140Cap.

9. Utilisation selon la revendication 7, **caractérisée en ce que** ledit composé pharmacologiquement actif interfère avec l'expression de la protéine p140Cap, de préférence ledit composé pharmacologiquement actif détermine une surexpression de la protéine p140Cap.

10. Utilisation selon l'une quelconque des revendications 1, 5 à 9, dans laquelle la tumeur est une tumeur solide, de préférence une tumeur du sein, de l'estomac, du côlon, de la prostate, de l'utérus, de l'ovaire, du pancréas.

11. Procédé de diagnostic ou de pronostic d'une croissance cellulaire anormale, le procédé consistant à mesurer l'expression du gène p140Cap dans un échantillon biologique issu d'un patient.

12. Procédé selon la revendication 11, **caractérisé en ce que** ledit procédé est un procédé immunochimique, un procédé d'hybridation d'ADN ou un procédé d'hybridation d'ARN.

13. Procédé selon la revendication 11 ou la revendication 12, **caractérisé en ce que** ladite croissance cellulaire anormale est une croissance cellulaire maligne.

14. Animal transgénique mammifère non humain, qui est transgénique en présentant une expression de p140Cap et de Neu-T altérée, où ladite expression de p140Cap altérée est obtenue par une modification stable de l'expression de p140Cap au niveau transcriptionnel, traductionnel ou post-traductionnel, où ladite expression de p140Cap altérée est une suractivation du gène p140Cap et où ledit animal transgénique mammifère non humain présentant une expression de p140Cap et de Neu-T altérée est généré en croisant un animal transgénique pour p140Cap avec un animal transgénique pour Neu-T.

15. Animal transgénique mammifère non humain selon la revendication 14, où ledit animal transgénique mammifère non humain appartient au genre murin (mus musculus).

16. Cellules pouvant être dérivées de l'animal transgénique mammifère non humain selon l'une quelconque des revendications 14 à 15 et présentant une expression de p140Cap et de Neu-T altérée.

17. Cellules selon la revendication 16, **caractérisées en ce que** lesdites cellules portent une mutation suractivant le gène p140Cap.
